# EUROPEAN PATENT APPLICATION

(11) **EP 4 628 130 A2**
(43) Date of publication of application: **08.10.2025**
(21) Application number: 25197916.7
(22) Date of filing: 19.10.2022
(51) Int. Cl.: A61M 15/00

(54) **BREATH-POWERED NASAL DEVICES FOR TREATMENT OF TRAUMATIC BRAIN INJURY (TBI), INCLUDING CONCUSSION, AND METHODS**

(30) Priority: 19.10.2021 US 202163257117 P
(62) Divisional of application: 22818508.8
(71) Applicant: Oragenics, Inc., Sarasota, FL 34236 (US)
(72) Inventor: VANLANDINGHAM, Jacob, Sarasota, 34236 (US); LEWANDOSKI, Michael, Sarasota, 34236 (US); STOWELL, Kelly, Sarasota, 34236 (US); LUCAS, Jonathan, Sarasota, 34236 (US); COCHRAIN, Travis, Sarasota, 34236 (US)
(74) Representative: Berggren Oy

(57) **Abstract**

The present invention is directed to a single-directional insufflator or breath-powered nasal device that provides unique dual airflow for propelling a drug substance into a nasal cavity, preferably deep into the superior nasal cavity and into the olfactory region and the trigeminal nerve mucosa, for rapid diffusion into the brain for the treatment of nasal and/or central nervous system ("CNS") injury, disease or disorder, especially brain injury, such as traumatic brain injury ("TBI"), including concussion, and methods regarding nasal treatment therewith.

Breath-powered nasal devices for delivering a somewhat tight or confined, concentrated plume of drug substance deep into the nasal cavity to targeted sites, bypassing the blood brain barrier, namely the olfactory nerve to provide concentrated drug substance for direct absorption into the brain are disclosed. The confined plume of drug substance propelled from the nasal device by a patient's breath is narrow in the vertical resembling the diameter of the inner chamber in which the drug substance is stored. By forming this narrow or confined plume, a greater concentration of the drug substance can be deposited at the deeper nasally targeted sites. The breath-powered nasal devices of the present invention accomplish this unique advantage by the use of a novel dual elongated double-walled tube chamber comprised of an outer hollow chamber and the inner chamber in which the inner chamber is stabilized in the outer chamber by preferably two, three or more ribs. More specifically, the breath-powered nasal device uses the patient's own breath to blow air simultaneously at the same force rate through both chambers. As the blown air exits both chambers, the propelled air from the outer tube chamber surrounds the drug substance propelled from the inner tube chamber within the blown air forcing the drug substance to exit in a tight or confined, concentrated plume shape in the vertical, so that a higher concentration of drug substance reaches the targeted nasal sites, as compared to a conical fanned-out plume shape of drug substance exiting a single hollow chamber, as illustrated in FIGS. 44-45. This unique feature allows diseases or injuries of the brain to be treated more effectively with a drug substance without or with minimized systemic or Blood Brain Barrier ("BBB") issue.

The present invention uses a patient's natural breath to blow air through both the inner drug chambers to propel a drug substance from the inner drug substance chamber deep into the nasal cavity to targeted sites, bypassing the BBB, providing the potential to better treat central nervous system diseases such as traumatic brain injury, including concussions, migraines, epilepsy, insomnia, and post-operative pain, the latter of which is often remedied by opioids.

## Description

### Related U.S. Provisional Application

This utility application for U.S. patent claims priority from U.S. provisional application, Serial Number 63/257,117, which was filed on October 19, 2021,of which the foregoing U.S. provisional application is incorporated herein by reference in its entirety as if fully set forth herein.

### Field of the Invention

The present invention is directed to a single-directional insufflator or breath-powered nasal device that provides unique dual airflow for propelling a drug substance into a nasal cavity, preferably deep into the superior nasal cavity and into the olfactory region and the trigeminal nerve mucosa, for rapid diffusion into the brain for the treatment of nasal and/or central nervous system ("CNS") injury, disease or disorder, especially brain injury, such as traumatic brain injury ("TBI"), including concussion, and methods regarding nasal treatment therewith.

### Background

The noninvasive nature of intranasal drug administration makes it an attractive option for local and systemic delivery of therapeutic agents. See FDA. Content current as of: 4/28/2021. How computational analysis of a 3D mucociliary clearance model can help predict drug uptake and lead to more generic nasal drug products. https://www.fda.gov/drugs/regulatory-science-research-and-education/how-computational-analysis-3d-mucociliary-clearance-model-can-help-predict-drug-uptake-and-lead-more ("FDA, 4/28/2021"). The nasal mucosa, unlike other mucosae, is easily accessible. FDA, 4/28/2021. Intranasal application circumvents the issues of gastrointestinal degradation and hepatic first-pass metabolism of drugs. FDA, 4/28/2021. See also Bitter C, Suter-Zimmermann K, Surber C.: Nasal drug delivery in humans. Karger Publishers, Topical Applications and the Mucosa. pages 20-35 (2011).

Drugs intended to hit a target site within the nasal cavity often get trapped in the highly viscous gel layer, which can reduce drug efficacy. FDA, 4/28/2021. Notwithstanding the trapping, soluble drugs dissolve in the mucus layer, diffuse across the gel and sol layers, and are eventually absorbed by the richly vascularized nasal epithelium. FDA, 4/28/2021. It is believed that this process within the nasal housing enables a drug to enter the systemic regions through the blood stream without losing efficacy. FDA, 4/28/2021.

For most nasal delivery purposes, a broad distribution of the drug on the mucosal surfaces appears acceptable for drugs that are intended for local action or systemic absorption. See, e.g., Vidgren MT, Kublik H.: Nasal delivery systems and their effect on deposition and absorption. Adv Drug Deliv Rev. 29:157-77 (1998). See also Djupesland, P.G.: Nasal drug delivery devices: characteristics and performance in a clinical perspective-a review. Drug Deliv. and Transl. Res. 3:42-62 (2013). In chronic sinusitis and nasal polyposis, however, strategic targeted delivery to the middle and superior meatuses at which the sinus openings are located and the polyps originate, is beneficial. See Laube B.: Devices for aerosol delivery to treat sinusitis. J Aerosol Med. 20(Suppl):5-18. 43 (2007); and Aggrawal R, Cardozo A, Homer JJ.: The assessment of topical nasal drug distribution. Clin Otolaryngol. 29:201-5 (2004). See also Djupesland, P.G.: Nasal drug delivery devices: characteristics and performance in a clinical perspective-a review. Drug Deliv. and Transl. Res. 3:42-62 (2013).

The above notwithstanding, there is an interest within industry for developing products that target blood-brain barrier (BBB) for rapid delivery to the central nervous system, including to the brain. Pardeshi CV, Belgamwar VS.: Direct nose to brain drug delivery via integrated nerve pathways bypassing the blood-brain barrier: an excellent platform for brain targeting. Expert opinion on drug delivery 10(7):957-72. (2013). See also FDA, 4/28/2021. Unfortunately, the most recent FDA guidance for nasal devices appears to essentially address *mechanical liquid spray pumps* and *pressurized metered-dose inhalers* for local action. FDA 2003. US FDA draft guidance for industry. Bioavailability and bioequivalence studies for nasal aerosols and nasal sprays. Bethesda. http://www.fda.gov/cder/guidance/index.htm. April 2003. See also Djupesland, P.G.: Nasal drug delivery devices: characteristics and performance in a clinical perspective-a review. Drug Deliv. and Transl. Res. 3:42-62 (2013) ("FDA 2003 Guidance"). See also Djupesland, P.G.: Nasal drug delivery devices: characteristics and performance in a clinical perspective-a review. Drug Deliv. and Transl. Res. 3:42-62 (2013). The FDA guidance apparently offers no guidance for breath-powered or breath-propelled nasal devices for nasal use for delivering drug substances into the brain for treatment of brain injury or disease. FDA 2003 Guidance. See also Djupesland, P.G.: Nasal drug delivery devices: characteristics and performance in a clinical perspective-a review. Drug Deliv. and Transl. Res. 3:42-62 (2013).

Unlike broad nasal drug substance distribution within the nasal cavity for local action or systemic absorption, drug substances intended for "nose-to-brain" (NTB) delivery present unique challenge. NTB drug delivery requires a more targeted delivery to the upper nasal parts located deep inside the nasal cavity of the nose housing for deposition into the olfactory region and onto the olfactory nerves which is essential for NTB drug delivery and effective brain treatment. Djupesland, P.G.: Nasal drug delivery devices: characteristics and performance in a clinical perspective-a review. Drug Deliv. and Transl. Res. 3:42-62 (2013).

Recent animal data suggest, however, that some degree of drug substance transport may occur along and across the branches of the first and second divisions of the trigeminal nerve innervating most if not all of the mucosa at and well beyond the nasal valve. Johnson H.J. et.al.: Trigeminal pathways deliver a low molecular weight drug from the nose to the brain and orofacial structures. Mol Pharm. (3):884-93 (2010). See also Djupesland, P.G.: Nasal drug delivery devices: characteristics and performance in a clinical perspective-a review. Drug Deliv. and Transl. Res. 3:42-62 (2013). Johnson H.J. *et al.* therefore suggests that a combination of targeted delivery to the olfactory region and a broad distribution to the mucosa innervated by the trigeminal nerve may be optimal for NTB delivery and effective brain treatment by a drug substance. Djupesland, P.G.: Nasal drug delivery devices: characteristics and performance in a clinical perspective-a review. Drug Deliv. and Transl. Res. 3:42-62 (2013).

The central nervous system (CNS) includes the brain, the brain stem, and the spinal cord. The CNS is isolated from the external world by several membranes that both cushion and protect the brain, the brain stem, and the spinal cord. For example, the membranes that form the blood-brain barrier (BBB) protect the brain from certain contents of the blood. The blood-cerebrospinal fluid barrier (BCSFB) protects other portions of the CNS from many chemicals and microbes. Thus, a two barrier system in brain forms the BBB, i.e., the brain microvascular endothelium and the choroid plexus epithelium (BCSFB). In addition to the BBB, there is an arachnoid epithelium barrier around the brain that creates the sub-arachnoid space.

Drug transport from blood to cerebrospinal fluid (CSF) is regulated by the choroid plexus, or BCSFB. Drug transport from blood to interstitial fluid (ISF) is regulated by the brain microvascular endothelium, or BBB. See W. M. Pardridge: Drug transport in brain via the cerebrospinal fluid. Fluids Barriers CNS. 8:7 (2011).

There is no lymphatic system in the human brain. Nevertheless, more than 500mls of cerebrospinal fluid is produced by the human brain every day. The CSF is secreted at the choroid plexus, or the BCSFB. The CSF fills the cranial and spinal sub-arachnoid space as well as the cavities of the four ventricles. See W. M. Pardridge: Drug transport in brain via the cerebrospinal fluid. Fluids Barriers CNS. 8:7 (2011). The ventricles of the brain are a communicating network of cavities occupied with the CSF and are located within the brain parenchyma. The ventricular system comprises two lateral ventricles, a third ventricle, a cerebral aqueduct, and a fourth ventricle. The choroid plexuses are located in the ventricles that secrete the CSF, which then fills the ventricles and subarachnoid space, following a cycle of constant production and reabsorption. See https://emedicine.medscape.com/article/1923254-
overview#: ~: text=Overview-, Overview,(see%20the%20images%20below).

The CSF flows over the surfaces of the brain and spinal cord. The CSF is rapidly absorbed into the general circulation. As discussed above, the choroid plexus forms the blood-cerebrospinal fluid barrier, and this barrier is functionally distinct from the brain microvascular endothelium, which forms the blood-brain barrier. Virtually all non-cellular substances in blood distribute into cerebrospinal fluid, and drug entry into cerebrospinal fluid is not an index of drug transport across the blood-brain barrier. Drug injected into the cerebrospinal fluid rapidly moves into the blood via bulk flow, but penetrates into brain tissue poorly owing to the limitations of diffusion. See W. M. Pardridge: Drug transport in brain via the cerebrospinal fluid. Fluids Barriers CNS. 8:7 (2011).

Conventional methods for delivering compounds to the CNS are generally invasive. For example, certain compounds, like epidural steroid injections (ESI), facet joint injections, hardware injections, sacroiliac (SI) joint injections and differential lower extremity injections, are injected directly through the protective membrane into the CNS. However, spinal injections are not trivial and are generally impractical for most compounds.

Another example for delivering a compound to the CNS is a pump implanted in the skull, such as an intracerebroventricular (ICV) pump, can deliver a variety of compounds to the brain. However, implanting such a pump requires brain surgery, which can entail a variety of serious complications. Nevertheless, ICV, or intraventricular, devices have been used in the treatment of a broad range of pediatric and adult central nervous system (CNS) disorders. Because of limited permeability of the blood brain barrier, CNS diseases generally require direct administration of drugs into the brain to achieve meaningful therapeutic effect. It is generally accepted that the clinical use and complications of ICV drug delivery result in device-associated complication rates as high as 33% for non-infectious complications and 27% for infectious complications. These undesirable safety outcomes may be due a lack of agreement amongst practioners on best practices of device use. See Slavc I. et al.: Best practices for the use of ICV drug delivery devices. Molecular Genetics and Metabolism. 124(3):184-188 (2018).

Much of current brain research is focused on the enhancement of the drug being delivered to the brain by various formulations. The traditional approaches to improve uptake of compounds to the brain include (1) mucoadhesive formulations; 2) penetration enhancers; 3) liposomes; 4) vasoconstrictors; and 5) nanoparticles. Examples of various compounds with have enhanced formulations include various cytokines, for example, tumor necrosis factors, interleukins, and interferons discussed in U.S. Pat. No. 6,991,785 and growth and differentiation factor-5 (GDF-5) and related proteins discussed in U.S. Publication No. 20100074959.

Targeting of drugs to the central nervous system (CNS) is a challenging task. A great number of drugs, including biotechnology products, are candidates for treatment of CNS diseases, but drug delivery through the BBB is a problem for brain targeting. A limitation in the treatment of brain tumors is that less than 1% of most therapeutic agents administered systemically are able to cross the BBB. The transport of small molecules across the BBB is the exception rather than the rule, and 98% of all small molecules do not cross the BBB. See, *e.g.,* Pardride, W.M: The blood-brain barrier: bottleneck in brain drug development. NeuroRx. 2(1):3-14 (January, 2005), in which Pardride concludes that approximately 100% of large-molecule drugs or genes do not cross the BBB. According to Pardride, the BBB allows small (about less than 500 Da), lipophilic molecules from the bloodstream to enter the CNS and that many larger therapeutic agents are prevented from reaching the brain for treating CNS disorders such as but not limited to Parkinson's disease, Alzheimer's disease, depression, stroke, and epilepsy. Pardride, W.M: The blood-brain barrier: bottleneck in brain drug development. NeuroRx. 2(1):3-14 (January, 2005). See also Chapman, C.D. et.al.: Intranasal Treatment of the Central Nervous System Dysfunction in Humans. Pharm. Res. 30:2475-2484 (2013).

Disorders including autism, lysosomal storage disorders, fragile X syndrome, ataxis, and blindness, are serious disorders where there is little effective treatment. In many of these cases, the gene underlying the disease is known, but BBB delivery is the rate-limiting problem in gene therapy or enzyme replacement therapy, and no therapeutics have been developed. Drug delivery of therapeutic compounds, for example proteins, faces several challenges because of their instability, high enzymatic metabolism, low gastrointestinal absorption, rapid renal elimination, and potential immunogenicity. Pardride, W.M: The blood-brain barrier: bottleneck in brain drug development. NeuroRx. 2(1):3-14 (January, 2005).

Pardridge believes that "... despite the importance of the BBB to the neurotherapeutics mission, the BBB receives insufficient attention in either academic neuroscience or industry programs and that combination of so little effort in developing solutions to the BBB problem, and the minimal BBB transport of the majority of all potential CNS drugs, leads predictably to the present situation in neurotherapeutics, which is that there are few effective treatments for the majority of CNS disorders." Pardride, W.M: The blood-brain barrier: bottleneck in brain drug development. NeuroRx. 2(1):3-14 (January, 2005). See also FDA 2003 Guidance.

According to Chapman, C.D. et.al., intranasal delivery is emerging as a noninvasive option for delivering drugs to the CNS with minimal peripheral exposure. Chapman, C.D. et.al.: Intranasal Treatment of the Central Nervous System Dysfunction in Humans. Pharm. Res. 30:2475-2484 (2013). See also FDA, 4/28/2021; Trevino, J.T. et al.: Non-Invasive Strategies for Nose-to-Brain Drug Delivery. J Clin Trials. 10(7):439. (2020); and Dhakar, R. C. et.al.: Nasal Drug Delivery: Success Through Integrated Device Development. J. Drug Deliv. Ther. 1(1): 2-7 (2011). Chapman, C.D. et.al. suggest that intranasal delivery facilitates the delivery of large and/or charged therapeutics, which fail to effectively cross the blood-brain barrier (BBB). Thus, Chapman, C.D. et.al. concludes that for a variety of growth factors, hormones, neuropeptides and therapeutics including insulin, oxytocin, orexin, and even stem cells, intranasal delivery is materializing as an efficient method of administration, and represents a promising therapeutic strategy for the treatment of diseases with CNS involvement, such as obesity, Alzheimer's disease, Parkinson's disease, Huntington's disease, depression, anxiety, autism spectrum disorders, seizures, drug addiction, eating disorders, and stroke. Chapman, C.D. et.al.: Intranasal Treatment of the Central Nervous System Dysfunction in Humans. Pharm. Res. 30:2475-2484 (2013).

However, intranasal administration has traditionally focused on the distribution of drug solutions as a mist for topical delivery to the nasal epithelium. Because of the nasal cavity's easily accessed vascular bed, nasal administration of medications has focused the delivery of medications either locally to the nasal cavity or directly to the blood stream.

There is a definite need for nasal insufflators that can deliver drug substances to the upper nasal cavity, thereby avoiding the BBB problems, for direct nose-to-brain or NTB delivery for effective treatment of brain injury and disorders. Certain existing nasal drug delivery devices do not adequately propel the drug from the device or concentrate the drug for effective targeting and treatment. Inconsistent propulsion of a steady flow of drug concentration due is also far from optimal. Still further, the plume generated by such existing devices is far too wide and scattered for effective NTB delivery. Certain existing nasal drug devices rely on circumferential velocity to propel medicaments to the olfactory epithelium. Traditional circumferential devices result in a lower percentage of drug substance being deposited on the olfactory epithelium. A circumferential component in the aerosol plume tends to result in a wider spray plume with a portion of the aerosol particles targeted to the sides of the nasal cavity in the lower part of the nasal cavity.

Thus, there is a definite clinical unmet need for improved nasal insufflators and mechanisms that effectively target and deliver concentrated depositions of desired drug substances within the brain, brain stem, and/or spinal cord and improve clinical performance and outcomes in the treatment of CNS and brain injury and disorders, such as TBI, especially concussions.

### Summary of The Invention

The present invention overcomes the above-mentioned disadvantages and drawbacks of the present state of nasal insufflator art through the discovery of a novel single-directional insufflator or breath-powered nasal device and methods that provide unique dual airflow for propelling a drug substance into a nasal cavity, preferably deep into the superior nasal cavity for effective targeted delivery to the olfactory region and distribution into the mucosa innervated by the trigeminal nerve in high drug substance concentration for rapid diffusion into the brain for the treatment of nasal and/or central nervous system ("CNS") injury, disease or disorder, especially brain injury, including traumatic brain injury ("TBI"), especially concussions.

The novel single-directional insufflator or breath-powered nasal devices and delivery methods of the present invention utilize both a novel dual wall drug dispensing system, i.e., a double walled-tube configuration, and the natural nasal anatomy and features to improve nasal delivery of drug substances in the form of fine particles, liquids, suspensions, gels and the like without pulmonary or gastric deposition or first-pass liver metabolism of the drug substance, and thus to improve nasal treatment efficacy. The novel single-directional insufflator or breath-powered nasal devices are preferably disposable, single-use devices for mono dose delivery of a single drug substance or for mono dose delivery of two or more drugs substances. The novel single-directional insufflator or breath-powered nasal devices of the present invention are hygienic and provide unique effective and accurate dosing of a drug substance for effective nasal treatment of local, systemic and CNS injury, disease or disorders.

Use of a novel single-directional insufflator or breath-powered nasal device of the present invention is intuitively simple and takes advantage of the natural nasal reflex for blowing a dry-powder or liquid dose deep into the nasal cavity for effective targeted delivery to the olfactory region and distribution into the mucosa innervated by the trigeminal nerve in high drug substance concentration for rapid diffusion into the brain. To illustrate, a subject using a device of the present invention snugly fits the nose guard piece on one end of the device into one nostril and then inserts or positions the mouth piece on the other end of the device into the mouth between the lips. When the subject exhales blown air through the inserted or positioned mouth piece against resistance generated from the device, the nasal reflex that automatically closes the soft palate is activated thereby closing the subject's oral and nasal airway passages. As a result, the blown drug substance loaded into the device is propelled from the nose guard, within the exiting unique dual airflow created by the novel dual wall drug dispensing system of the device, and is delivered and trapped deep within the nasal cavity for delivering the trapped drug substance to the superior nasal membranes within the olfactory region and the trigeminal nerve mucosa for diffusion directly into the brain within minutes of device use. Once the subject's blow for dose delivery to the nasal cavity is complete, the subject's nasal reflex naturally subsides and the subject's oral and nasal airway passages re-open.

While any effective blown airflow rate is contemplated by the present invention, a blown airflow rate of about 40 Liters per Minute at a pressure of at least about 40 cm H₂O pressure or greater appears to be more than suitable to effectively operate a novel single-directional insufflator or breath-powered nasal device of the present invention.

It is therefore among objectives of this invention to provide a novel single-directional insufflator or breath-powered nasal device intended for treatment of local, systemic and CNS injury, disease and disorders, especially TBI and more especially concussion, through the use of a novel and unique combination of dual airflow to propel highly concentrated drug substance deep into a nasal cavity for deposition into and distribution throughout the olfactory region and distribution into the mucosa innervated by the trigeminal nerve and for diffusion directly into the brain thereby uniquely avoiding systemic and BBB issues. The novel single-directional insufflator or breath-powered nasal device may, if so elected, also deposit a drug substance within the nasal cavity for local and systemic application of the same or different drug substances. These objectives are accomplished by the novel and unique dual wall drug dispensing system configured within the novel single-directional insufflator or breath-powered nasal device of the present invention.

Generally speaking, the single-directional insufflator or breath-powered nasal devices of the present invention comprises a one-way valve mouth piece at one end and a nose guard at the other end that are fluidly connected via a corrugated tubing for delivering a loaded drug substance deep within the nasal cavity through oral pressurization by blowing through the one-way valve mouth piece and through the novel dual-wall drug-substance technology to cause the drug substance to exit the nose guard in a vertical, somewhat tight and narrow or confined, concentrated plume for delivering a high drug substance concentration to the targeted sites deep within the nasal cavity and distributing throughout the olfactory region and into the mucosa innervated by the trigeminal nerve and for diffusing therefrom into the brain. Delivery of the drug substance, in for example, dry powder, liquid, suspension or gel form and the like, occurs when the nose guard is inserted snugly into one nostril of a person and the person blows into the one-way valve mouth piece, which breath closes the person's velum (soft palate) and causes the pressurized airflow to travel through the one-way valve mouth piece and then through the novel dual wall drug dispensing system of the present invention, which upon nose guard exit, uniquely carries the drug substance in the vertical, somewhat tight or confined and narrow, concentrated plume shape in high drug concentration deep into the superior nasal cavity for effective distribution in the olfactory region and the mucosa innervated by the trigeminal nerve and for direct diffusion therefrom into the brain.

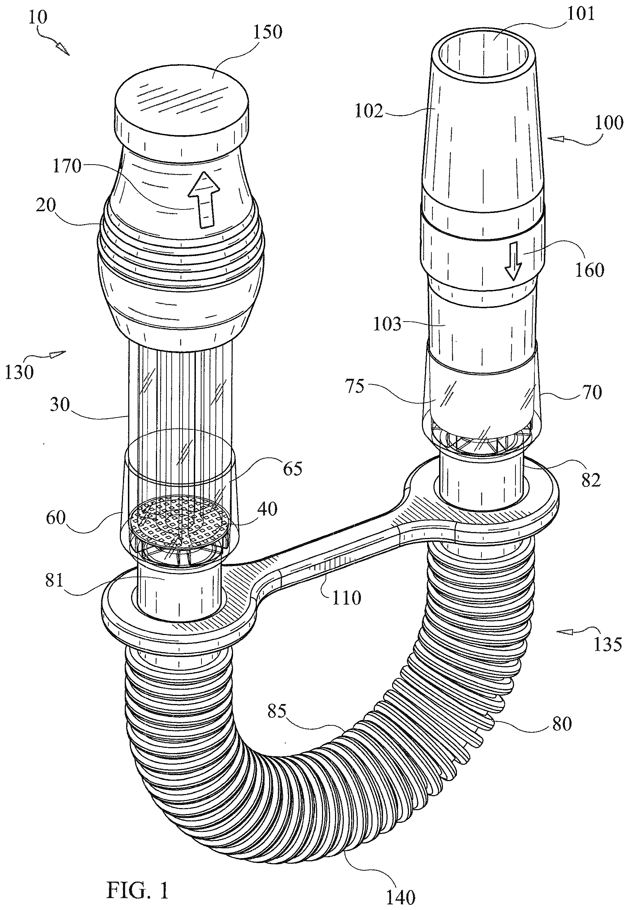

More specifically, and as shown in **Figs. 1-2** and **9-10,** single-directional insufflator or breath-powered nasal device **10** of the present invention comprises nose guard **20,** dual wall drug dispensing system **30,** mesh disc **40** on which drug **200** (not shown) is positioned, first tube connector **60,** corrugated tubing **80,** second tube connector **70,** ridge connector **110,** one-way valve mouth piece **100** housing a one-way valve **50** (not shown) in bottom section **103** that directs airflow in one direction and prevents reverse airflow direction, so that, first air-flow directional indicator band **160** and second air-flow directional indicator band **170,** when single-directional insufflator or breath-powered nasal device **10** is in use and seal cap **150,** i.e., top cover, is removed and user blows air through the one-way vale mouth piece **100,** drug **200** (not shown) positioned on mesh disc **40** is propelled out of nose guard **20.**

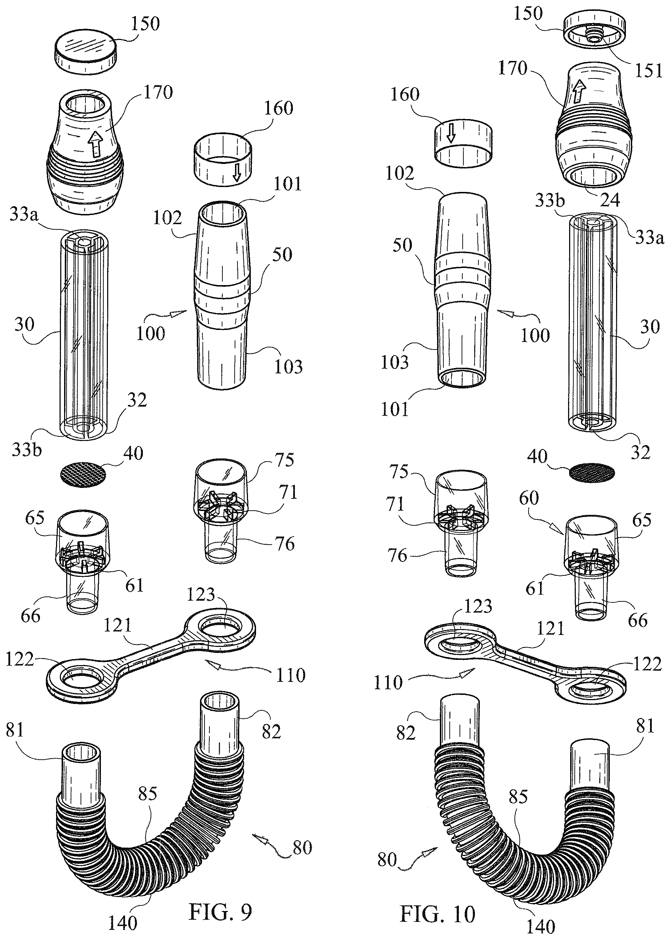

As further shown in **Figs. 9-10****,** the top end portion **31**of the dual wall drug dispensing system **30** is press-fit through bottom **26** of through-hole **24** of nose guard **20** and positioned snugly against nose guard seat **22** located near top **25** of through-hole **24** of nose guard **20.** The bottom end portion **32** of the dual wall drug dispensing system **30** is press-fit into first top hollow collar **65** of first tube connector **60** to form a tight seal there between. Mesh disc **40** is inserted into and sits on top of first seat **61** located at the bottom of first top hollow collar **65** of first tube connector **60.** First bottom hollow neck **66** of first tube connector **60** is press-fit into the first hollow cuff **81**of corrugated tubing **80** to form a tight seal there between. Second bottom hollow neck **76** of second tube connector **70** is press-fit into the second hollow cuff **82** of corrugated tubing **80** to form a tight seal there between. Bottom section **102** of one-way valve mouth piece **100** is press-fit into second top hollow collar **75** of second tube connector **70** until it is resting on second seat **71** of second tube connector **70** to form a tight seal there between.

Thus, and as further shown in **Figs. 9-10****,** one-way valve **50** (not shown) housed in bottom section **103** of one-way valve mouth piece **100** directs air-flow in one direction (prevents reverse air-flow direction) through (a) conduit **101** in one-way valve mouth piece **100,** (b) second bottom hollow neck **76** of second tube connector **70,** (c) corrugated tubing **80,** (d) first bottom hollow neck **66** of first tube connector **60** and mesh disc **40** sitting on first seat **61** in first tube connector **60,** (e) inner and outer hollow chambers **33a** and **33b** of dual wall drug dispensing system **30** that create novel inner and outer air-flows in which drug substance **200** is concentrated within and propelled by the inner air-flow and the outer air-flow surrounds the inner air-flow to concentrate drug substance **200** within inner air-flow as it exits top end portion **31** of inner hollow chamber **33a** and top **25** of through-hole **24** (not shown) of nose guard **20,** so that drug substance **200,** when breath-powered, is propelled from the novel, single-directional insufflator or breath-powered nasal device **10**meshmeshmeshmesh within a novel and unique double air-flow in a vertical, somewhat tight or confined and narrow, concentrated plume in high drug concentration, i.e., the inner air-flow, deep into the superior nasal cavity for effective distribution into the olfactory region and the mucosa innervated by the trigeminal nerve and for direct diffusion therefrom into the brain that is driven by the blown air through the novel, the single-directional insufflator or breath-powered nasal device **10** .

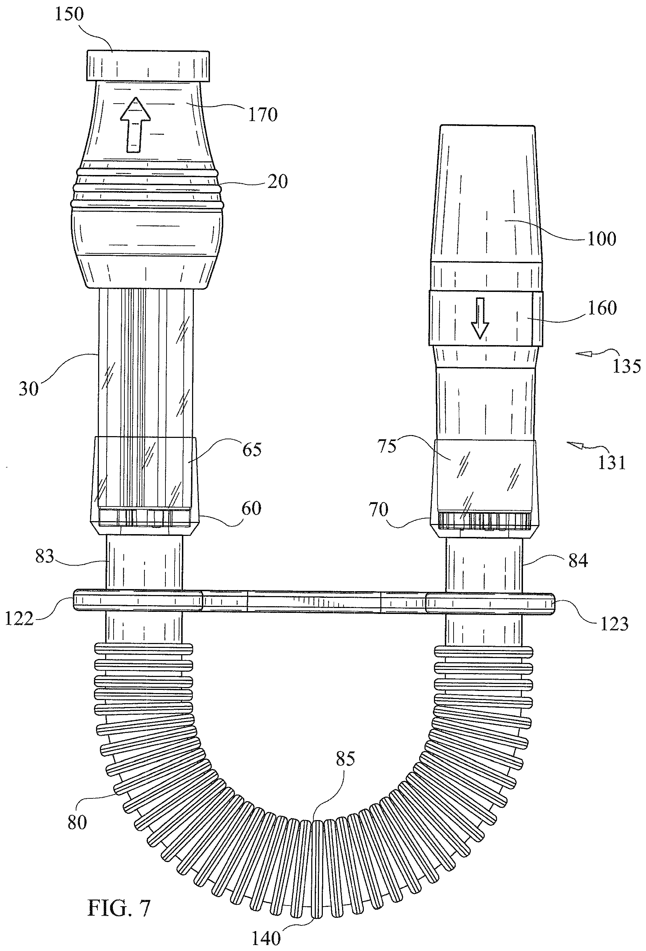

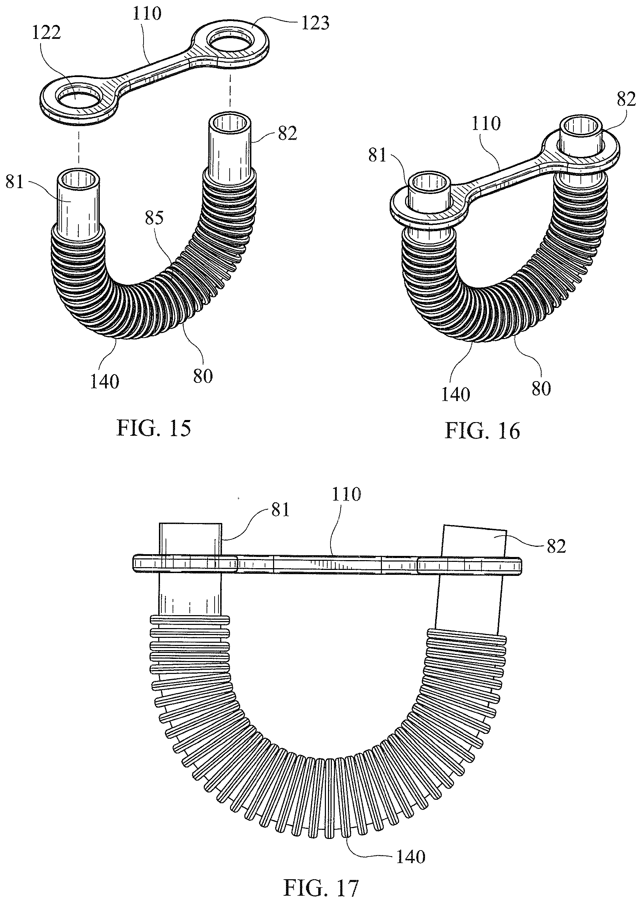

As depicted in **Figs. 7-8****,** a bridge connector **110** connects one side **131** of the novel, the single-directional insufflator or breath-powered nasal device **10** comprising the one-way valve mouth piece **100** and the second tube connector **70** to the other side **131** of the novel, the single-directional insufflator or breath-powered nasal device **10** comprising the nose guard **20,** the dual wall drug dispensing system **30,** the mesh disc **40** and the first tube connector **60** to maintain breath-powered insufflator or breath-powered nasal device **10** of the present invention in a "U-shape" configuration **140.** Bridge connector **110** comprises bridge connector **110** sandwiched between a first hollow ring **122** and a second hollow ring **123** for sliding over the first hollow cuff **81** and second hollow cuff 82 of corrugated tubing **80.** See **FIGS. 15-16** and **18-19.**

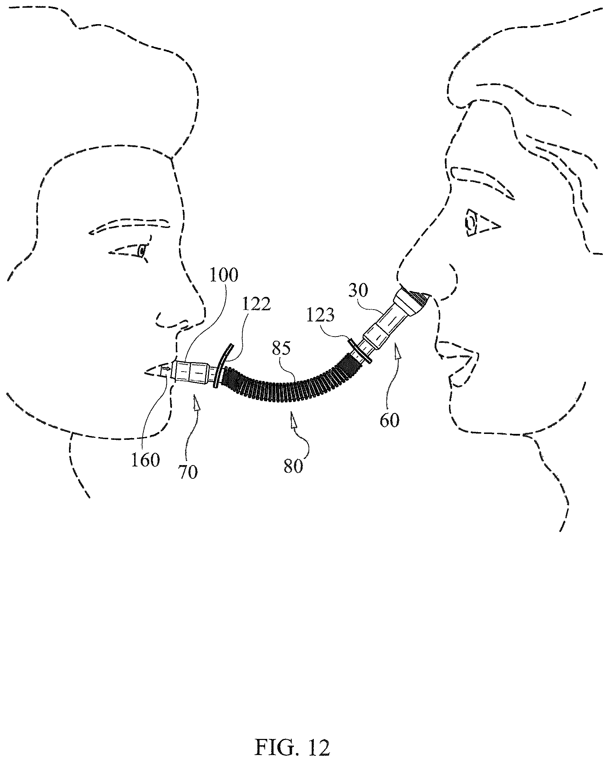

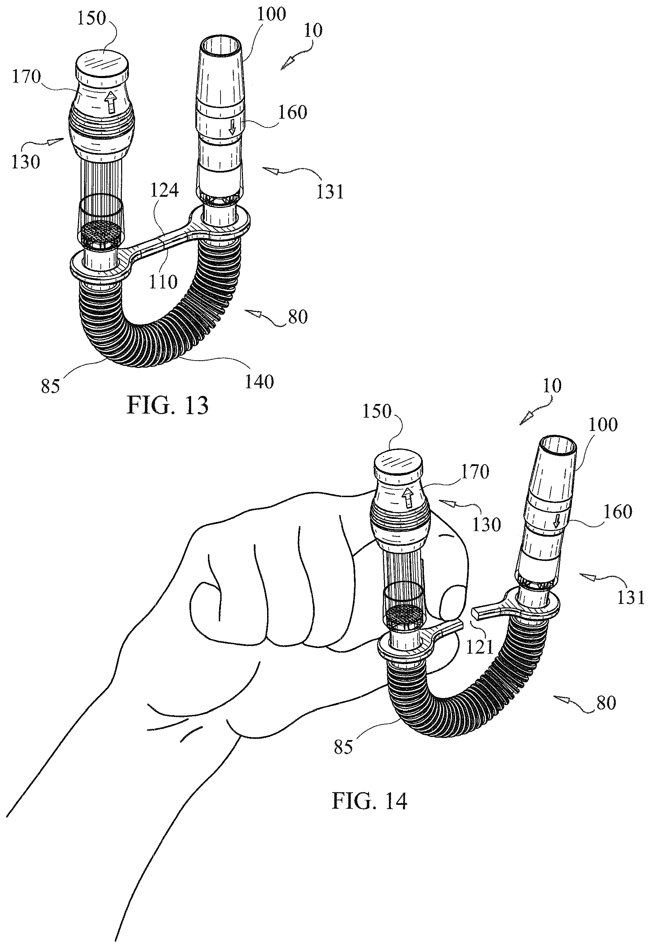

Alternatively, and as shown **Figs. 13-14****,** bridge connector **110** affixed to single-directional insufflator or breath-powered nasal device **10,** illustrated in front view **135** with nose guard side **130** and one-way valve mouth piece side **131,** may be designed with a perforation or score line **124** or any other configuration that allows bridge connector **110** to be broken and separated into two individual pieces in the event sides **130** and **131** of breath-powered insufflator or breath-powered nasal device **10** need to be orientated or adjusted at a different angle for use by, for example, a concussed person, or a second person who is assisting a person who, for example, is a concussed person, who cannot self-administer the necessary blow force to provide sufficient airflow to effectively propel the drug substance from the single-directional insufflator or breath-powered nasal device **10** of the present invention to effectively treat the concussion with the drug substance, as shown in **FIG. 12****.**

Dual wall drug dispensing system **30,** i.e., Dual-walled tubing configuration, is a novel and unique delivery system for delivering one or more drug substances into the nasal cavity. The dual wall drug dispensing system **30** comprises a top end portion **31** and the bottom end portion **32** and two hollow chambers **33,** an inner hollow chamber **33a** and an outer hollow chamber **33b.** The drug substance **200** is propelled through hollow inner chamber **33a** when the breath-powered insufflator or breath-powered nasal device **10** of the present invention is breath propelled. The hollow outer chamber **33b,** which surrounds the hollow inner chamber **33a,** is divided into two, three, four or more separate chambers **34** by rib walls **35.** Preferably, but not necessarily critical, hollow outer chamber **33b** is divided into three separate chambers **34.** In addition to providing multiple hollow chambers **34,** rib walls **35** impart integrity, structure and strength to and support for dual wall drug dispensing system **30.**

While the present invention contemplates dual chamber **34** comprised of inner hollow chamber **33a** and outer hollow chamber **33b** of the dual wall drug dispensing system **30,** i.e., Dual-walled tubing configuration, can be of any suitable dimensions necessary to perform effectively the objectives of the present invention, it is preferred that inner hollow chamber **33a** can accommodate up to about 120 mg or more and any amount there between of drug substance in dry powder or other form and its outer wall diameter **331** can range from, for example, about 3mm to about 7mm or more or less and its inner wall diameter **332** can range from about 2mm to about 5mm or more or less, and outer wall diameter **333** can range from, for example, about 9 mm to about 13mm or more or less and its inner wall diameter **334** can range from about 7mm to about 12mm or more or less.

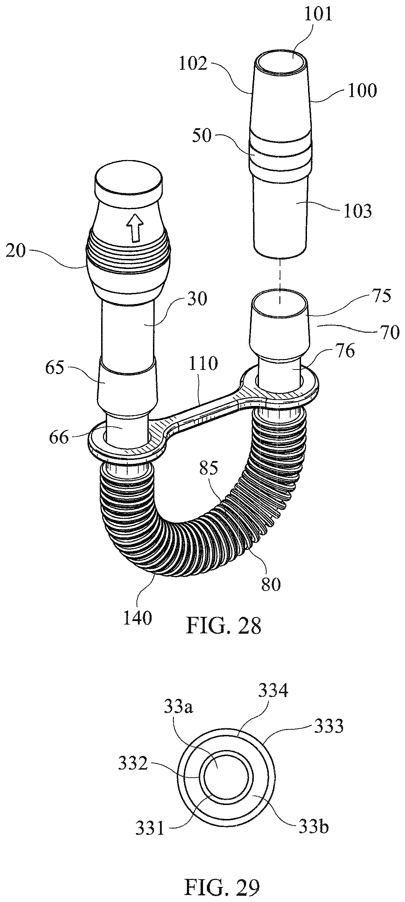

Preferably, and by way of example, as shown in **FIG. 29****,** one inner hollow chamber **33a** contemplated by the present invention is dimensioned with an outer wall **331** diameter of about 5mm and an inner wall **332** diameter of about 3mm creating an internal gap **37** diameter of inner hollow camber **33a** of about 3 mm. While these exemplary diameter dimensions generate about a 3mm internal gap **37** between outer wall 333 of inner hollow chamber **33a** and inner wall 334 of outer hollow chamber **33b,** the present invention also contemplates inner hollow chambers **33a** designed with an internal gap **37** diameter of up to about 1mm to about 4mm or greater. Thus, in accordance with the present invention, and preferably, internal gap **37** diameters may range from about 1mm to about 4mm or more, including internal gap 37 diameters of about 1mm, about 1.5mm, about 2mm, about 2.5mm, about 3mm, about 3.5mm and about 4mm and dimension sizes there between, so that nose guard **20** can of a size that it allows nose guard **20** can be sized to snuggly accommodate any size nostril as discussed herein before.

While the thicknesses of outer wall **331** and inner wall **332** of inner hollow chamber **33a** and outer wall **333** and inner wall **334** of outer hollow chamber **33b** may be of any suitable thickness, it is preferred that the actual thickness of walls, **331, 332** and **333, 334** of the dual wall drug dispensing system **30,** i.e., dual-walled tubing configuration, is about 2 mm thick.

To load the drug substance **200,** when in a powdered, spray-dried format, into a novel single-directional insufflator or breath-powered nasal device **10** of the present invention, a sterilized funnel (not shown) is used to load the powdered drug substance **200** specifically within inner hollow chamber **33a** of dual wall drug dispensing system **30.** The mesh disc **40,** which is positioned at bottom end portion **32** of inner hollow chamber **33a** and on first seat 61 of first tube connector as shown in **FIGS. 9-10** and **23,** is what the loaded powdered drug substance **200** first encounters and against which it rest once fully loaded. Because the pore size of mesh disc **40** is smaller than the particle size of the powdered drug substance, the powdered drug substance cannot fall through mesh disc **40.** The pores throughout mesh disc **40,** however, permit the blown airflow to penetrate up and through mesh disc **40** to carry and propel powdered drug substance **200** through the inner hollow chamber **33a** of dual wall drug dispensing system 30, i.e., the double walled-tube configuration, and top **25** of through-hole **24** and out of nose guard **20** deeply into the nasal cavity. It should be understood that if a liquid, suspension, gel or the like is loaded into dual wall drug dispensing system **30,** i.e., the double walled-tube configuration, a different mesh disc **40** that prevents leakage or seepage there through may be warranted.

Preferably, the particle size distribution of powdered, spray-dried, drug substance **200** is principally in the range of about 1µm to about 10µm or more. In one embodiment, the powdered, spray-dried, drug substance **200** contains a medicament, particularly for the treatment of a nasal condition. Notwithstanding the preferred particle size, the present invention contemplates any effective particle size distribution of a powdered, spray-dried, drug substance that will accomplish the objectives of the present invention, including larger particles, or a smaller fraction of larger particles, typically in the range of about 10µm to about 30µm, and preferably in the range of about 20µm to about 30µm.

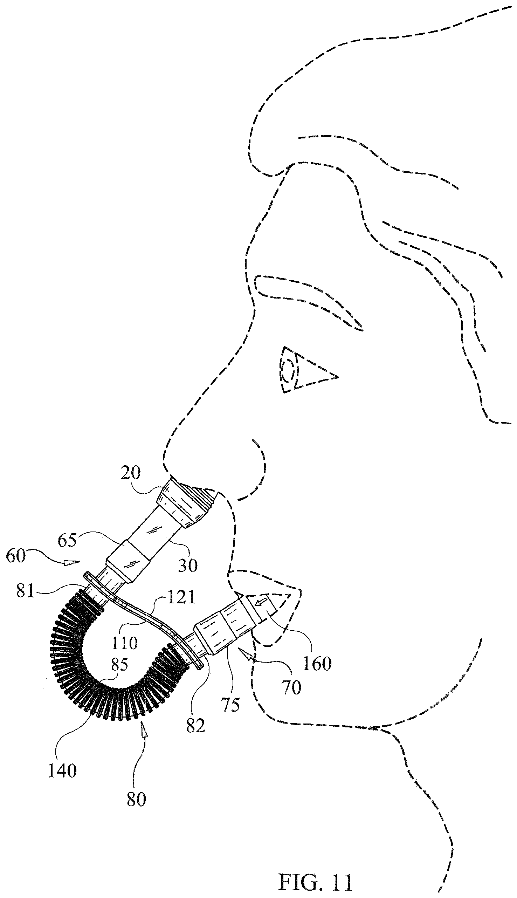

Turning now to operation, when a person blows or exhales through mouth piece **100** of the single-directional insufflator or breath-powered nasal device **10** of the present invention, as shown in **FIGS. 11** and **12****,** the blown air flows through both hollow chambers **33a** and **33b** of dual wall drug dispensing system **30,** i.e., the double walled-tube configuration, simultaneous and with essentially the same force expelling the drug substance loaded into inner hollow chamber **33a** from nose guard **20.** The air-flow pressure exiting from outer hollow chamber **33b** surrounds the airflow burdened with high drug substance concentration exiting from inner hollow chamber **33a** forcing the drug substance loaded air-flow to travel at least initially in a vertical, somewhat tight or confined and narrow, concentrated plume carrying high drug substance concentration into the deepest part of the superior nasal cavity, namely, the olfactory region and nerve and the mucosa innervated by the trigeminal nerve for direct diffusion therefrom into the brain. Thus, novel dual wall drug system **30,** i.e., a dual wall-tube configuration, uniquely and surprisingly delivers high drug substance concentration to the targeted olfactory region, even the mucosa innervated by the trigeminal nerve, for direct diffusion into the brain for effective treatment of CNS injury, disease and/or disorders, including the brain, the brain stem, and the spinal cord and, in particular TBI, especially concussion.

While it should now be appreciated that the unique design of the single-directional insufflator or breath-powered nasal device **10** of the present invention uniquely targets drug deposition at and into the olfactory region and the trigeminal nerve mucosa for direct diffusion into the brain, the initial flow pattern of the exiting drug substance loaded airflow may fan out to some extent causing some drug substance to be deposited on other parts of the nasal cavity. It should be further appreciated that, while it is preferred to load only the inner hollow chamber **33a** with drug substance **200,** different or same drug substance(s) may be loaded into the two or more separate chambers **34** of outer hollow chamber **33b** for deposition of drug substance(s) throughout the nasal cavity to treat CNS and nasal cavity and other nasal disorders treated via nasal application.

Thus, the present invention is uniquely suited to treat local, systemic and CNS injuries and/or disorders. It should be understood, however, that the single-directional insufflator or breath-powered nasal device **10** of the present invention is preferably a single use, disposable device especially designed to treat TBI, preferably mild, moderate and severe TBI, more preferably, mild to moderate TBI, even more preferably mild TBI, and most preferably, concussion. An example of drug substances suitable for use in combination with the single-directional insufflator or breath-powered nasal device **10** are C-20 steroid compounds in dry powder, spray-dried form, as disclosed and described in U.S. Patent Publication No. 2016/0168190, entitled "C-20 steroid compounds, compositions and uses thereof," and WO2016/044559A1, entitled "C-20 steroid compounds, compositions and uses thereof to treat traumatic brain injury (TBI), including concussions," each of which is incorporated herein by reference in its entirety as if it was fully set forth herein. One preferred C-20 steroid compound that is contemplated by the present invention is *ent-*19-norprogesterone in dry-powder, spray-dried form. *Ent*-19-norprogesterone has a molecular formula of C₂₀ H₂₈ O₂ and a molar mass of 300.435 g/mol. One chemical name for *ent*-19-norprogesterone is *ent*-19-norpregn-4-ene-3,20-dione. The chemical structure of *ent-*19-norprogesterone is as follows:

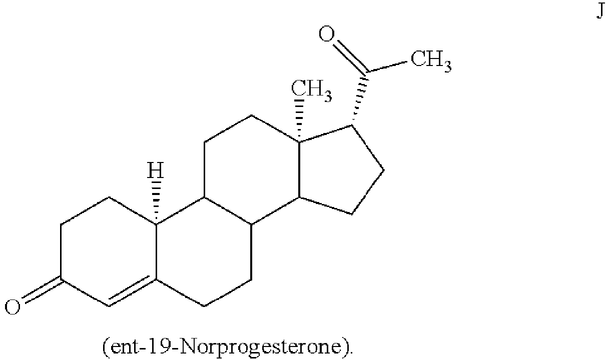

*ent-*19-norprogesterone is a fully synthetic, non-naturally, occurring neurosteroid. In preclinical studies, *ent-*19-norprogesterone has demonstrated equivalent, if not superior, neuroprotective effects compared to related neurosteroids. Animal models of concussion demonstrated that *ent-*19-norprogesterone reduces the behavioral pathology associated with brain injury symptoms such as memory impairment, anxiety, and motor/sensory performance. Additionally, *ent-*19-norprogesterone is lipophilic and can easily cross the blood-brain barrier to rapidly eliminate or at least reduce swelling, oxidative stress and inflammation in the brain while the healing process from TBI, e.g., concussion, attempts to restore proper blood flow and heal damaged or affected brain and/or other tissue.

When *ent-*19-norprogesterone is the drug substance of choice to treat TBI, especially concussion, inner hollow chamber **33a** is pre-loaded in accordance with the present invention with preferably about 100 mg of drug substance *ent*-19-norprogesterone, wherein the *ent*-19-norprogesterone drug substance comprises about 8 mg and the pharmaceutical acceptable excipient, hydroxypropyl-β-cyclodextrin, comprises about 92 mg. This 19-norprogesterone formulation is preferably in dry-powder, spray-dried format and can be administered up to four or more times a day for as long as necessary in accordance with a prescribed treatment regimen to treat, for example, a patient suffering with TBI, especially a concussion. To administer *ent-*19-norprogesterone drug substance preloaded into inner hollow chamber **33a** of the dual wall drug dispensing system **30** of the single-directional insufflator or breath-powered nasal device **10** of the present invention, the concussed or TBI injured person inserts nose guard **20** into one nasal vestibule or nostril until it is snug-fitting and then inserts mouth piece **100** between his/her lips. Once the single-directional insufflator or breath-powered nasal device **10** of the present invention is properly fitted in the nostril and between the lips of the concussed or TBI injured person, the concussed or TBI injured patient blows/exhales through mouth piece **100:** (a) to close the velum or soft palate to isolate the nasal cavity to prevent the *ent-*19-norprogesterone drug substance from being inhaled or passing into the digestive system and therefore reducing systemic side effects; and (b) to propel the *ent-*19-norprogesterone drug substance in the inner hollow chamber **33a** from the nose guard **20** with sufficient airflow force and pressure, that concentrated *ent*-19-norprogesterone drug substance is deposited onto and distributed throughout the olfactory region and the trigeminal nerve mucosa for direct diffusion into the brain for effective treatment of the concussion or TBI within minutes of nasal application. Importantly, one-way valve mouth piece **100** prevents reverse air-flow and the closed soft palate will trap the concentrated *ent-*19-norprogesterone drug in the nasal cavity allowing it to stick to the superior nasal membranes for diffusion directly into the brain by way of the olfactory nerve and the trigeminal nerve mucosa.

This very advantageous and unique nasal delivery is accomplished by the novel dual wall drug dispensing system **30,** i.e., the double walled-tube configuration, of the single-directional insufflator or breath-powered nasal device **10** of the present invention through the use of the unique dual air-flow combination of (i) a first air-flow expelled from the inner hollow chamber **33a** carrying the *ent-*19-norprogesterone drug substance, and (ii) a second air-flow expelled simultaneously from the outer hollow chamber **33b** to create air-flow pressure that surrounds the first expelled air-flow, so that the first expelled air flow forms and travels initially in a vertical, somewhat tight or confined and narrow, concentrated plume that drives a concentrated drug substance, e.g., *ent*-19-norprogesterone drug substance, to the targeted olfactory region, including the olfactory nerve, and the trigeminal nerve mucosa for direct diffusion into the brain and for effective treatment of the concussion or TBI within minutes of nasal application.

While single-directional insufflator or breath-powered nasal device **10** of the present invention is simple in construction, the operation of the device does provide for the effective delivery of drug substances, in particular one of powder containing the drug substance, to the posterior region of the nasal airway, since the powdered drug substance is delivered in a single-direction deep into the nasal cavity and the air-flow into the nasal cavity is through the same opening, namely, the respective nostril, with the closed posterior region of the respective nasal cavity acting as a pressure reflecting surface which causes the exhaled air to adequately reach the posterior region of the respective nasal cavity for deposition into the olfactory region and the trigeminal nerve mucosa for rapid diffusion into the brain for the treatment of nasal and/or CNS injury, disease or disorder, especially brain injury, such as TBI, including concussion. Further, in providing a short explosive burst of air-flow into one of the nasal cavities, it is now possible to achieve a sustained single-directional air-flow deep within the nasal cavity of high concentrated powdered drug substance, which is believed to be necessary to deliver a powdered drug substance effectively to the posterior region of the nasal airway for deposition into the olfactory region and the trigeminal nerve mucosa for rapid diffusion into the brain.

As contemplated by the present invention, any suitable materials may be used for the individual parts or components of the single-directional insufflator or breath-powered nasal devices **10** of the present invention, VESTAMID ML21 is an example of a selected material for plastic parts, e.g., dual wall drug dispensing system **30** and tube connectors **60** and **70.** ABS is an exemplary material for mouth piece **100** and one-way valve **50** (not shown). PTFE is an exemplary material for corrugated tubing **80.** Silicone HD-150A is exemplary material for nose guard **20,** bridge connector **110** and seal cap **150,** i.e., top cover.

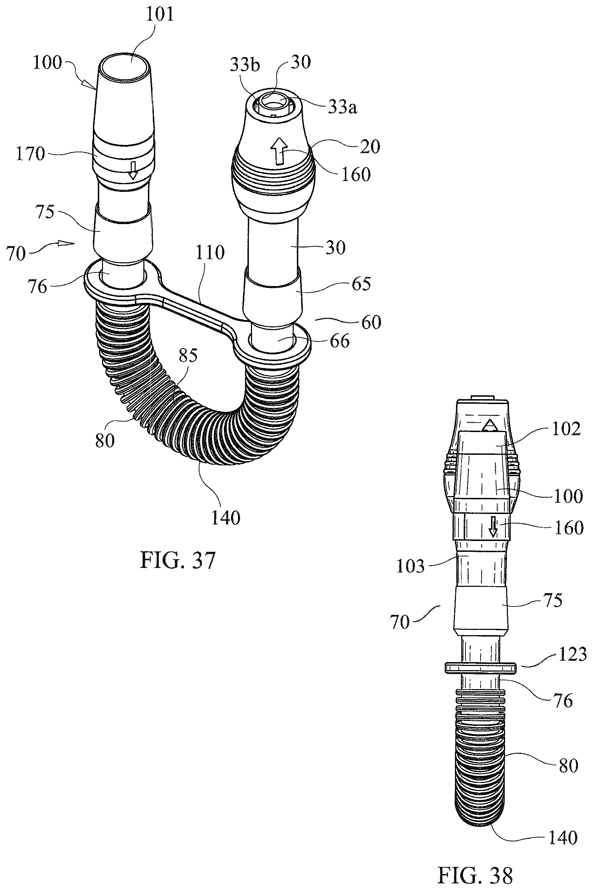

In accordance with one embodiment of the present invention, the length of the dual wall drug dispensing system **30,** i.e., the double walled-tube configuration, is about 2.00 inches and the length of the corrugated tube is about 4 inches. Total length of such an exemplary device is about 8 inches. When in U-shape configuration, the maximum length of such device is about 4 inches from the bottom of the corrugated material to the top of the nose guard **20** and the width is about 2.2 inches. The width of the corrugated material from side view is about 0.45 inches and the widest width of nose guard **20** is about 0.75 inches, as shown in **FIG. 38****,** and the widest width of one-way valve mouth piece **100** is about 0.5 inches.

It should be further understood that the above summary of the present invention is not intended to limit or describe each and every possible embodiment or implementation of the present invention, but contemplates such possible embodiments and implementations. The description, however, does exemplify illustrative embodiments. In several places throughout the specification, guidance is provided through examples, which examples can be used in various combinations. In each instance, the examples serve only as representative groups and should not be interpreted as exclusive examples.

### Description of the Figs.

The structure, operation and advantages of this invention will become further apparent upon consideration of the following description, taken in conjunction with the accompanying Figs., wherein:
**FIG. 1** is a front assembled elevational angled front view **135** of a single-directional insufflator or breath-powered nasal device **10,** in accordance with an embodiment of the of the present invention;
**FIG. 2** is a rear assembled elevational angled back view **136** of a single-directional insufflator or breath-powered nasal device **10,** in accordance with an embodiment of the of the present invention;
**FIG. 3** is a top view **850** of a corrugated tube **80** showing corrugated middle section **85** and bridge connector **110,** in accordance with an embodiment of the of the present invention;
**FIG. 4** is a bottom view **860** of a corrugated tube **80** showing corrugated middle section **85,** in accordance with an embodiment of the of the present invention;
**FIG. 5** is an assembled elevational view of nose guard side **130** of the single-directional insufflator or breath-powered nasal device, in accordance with an embodiment of the of the present invention;
**FIG. 6** is an assembled elevational view of the one-way valve mouth piece side **131** of the single-directional insufflator or breath-powered nasal device, in accordance with an embodiment of the of the present invention;
**FIG. 7** is a right mouth piece assembled elevational view of a single-directional insufflator or breath-powered nasal device, in accordance with an embodiment of the of the present invention;
**FIG. 8** is a left mouth piece assembled elevational view of a single-directional insufflator or breath-powered nasal device, in accordance with an embodiment of the of the present invention;
**FIG. 9** is a right mouth piece front unassembled exploded view of a single-directional insufflator or breath-powered nasal device, in accordance with an embodiment of the of the present invention;
**FIG. 10** is a left mouth piece front unassembled exploded view of a single-directional insufflator or breath-powered nasal device, in accordance with an embodiment of the of the present invention;
**FIG. 11** schematically illustrates a single-directional insufflator or breath-powered nasal device of **FIGS. 1-2** and **4-8** inserted into a nasal cavity of a subject for operation, in accordance with an embodiment of the of the present invention;
**FIG. 12** schematically illustrates a nose guard of an alternative embodiment of a single-directional insufflator or breath-powered nasal device as shown in **FIGS. 13** and **14** inserted into a nasal cavity of a first subject to be treated with a drug substance and a mouth piece of the alternative embodiment as shown in **FIGS. 13** and **14** inserted between the lips of a second subject for operation of the alternative embodiment by the second subject as shown in **FIGS. 13** and **14****,** in accordance with an embodiment of the of the present invention;
**FIG. 13** is a front, right mouth piece, assembled elevational angled view of an alternative embodiment of a single-directional insufflator or breath-powered nasal device herein wherein the bridge connector is designed with a perforated or score line for breaking the bridge connector, in accordance with an embodiment of the of the present invention;
**FIG. 14** is a front, right mouth piece, assembled elevational angled view of an alternative embodiment of a single-directional insufflator or breath-powered nasal device herein wherein the bridge connector is broken in two pieces at the perforated or score line for breaking the bridge connector, as shown in **FIG. 13****,** in accordance with an embodiment of the of the present invention;
**FIG. 15** schematically illustrates assembling bridge connector **110** to corrugated tubing **80,** in accordance with an embodiment of the of the present invention;
**FIG. 16** schematically illustrates a perspective view of bridge connector **110** and corrugated tubing **80** assembled in accordance with an embodiment of the of the present invention;
**FIG. 17** schematically illustrates a front view of bridge connector **110** and corrugated tubing **80** assembled in accordance with an embodiment of the of the present invention;
**FIG. 18** schematically illustrates assembling first tube connector **60** and second tube connector **70** to corrugated tubing **80** assembled with bridge connector **110,** as shown in FIGS. **16** and **17****,** in accordance with an embodiment of the of the present invention;
**FIG. 19** schematically illustrates a perspective view of first tube connector **60** and second tube connector **70** assembled with corrugated tubing **80** assembled with bridge connector **110,** as shown in **FIGS. 16** and **17****,** in accordance with an embodiment of the of the present invention;
**FIG. 20** schematically illustrates a cross-sectional view of a tube connector, in accordance with an embodiment of the of the present invention;
**FIG. 21** schematically illustrates inserting a mesh disc **40** into first tube connector **60,** wherein first tube connector **60** and second tube connector **70** are assembled with corrugated tubing **80** assembled with bridge connector **110,** as shown in **FIGS. 16** and **17****,** in accordance with an embodiment of the of the present invention;
**FIG. 22** schematically illustrates inserting a dual wall drug dispensing system **30,** i.e., a double-walled tube configuration, into first tube connector **60,** wherein mesh disc **40** is inserted into first tube connector **60** and first tube connector **60** and second tube connector **70** are assembled with corrugated tubing **80** assembled with bridge connector **110,** as shown in **FIGS. 16, 17****,** **19** and **21** , in accordance with an embodiment of the of the present invention;
**FIG. 23** schematically illustrates an exploded cross-sectional view of assembled first tube connector **60,** wherein mesh disc **40** and dual wall drug dispensing system **30,** i.e., a double-walled tube configuration, are assembled within first tube connector **60** and mesh disc **40** is sitting on first seat **61** of first tube connector **60** and first top hollow collar **65** of first tube connector **60** is press-fit into first hollow cuff **81** of corrugated tubing **80,** in accordance with an embodiment of the of the present invention;
**FIG. 24** schematically illustrates inserting top end portion **31** of dual wall drug dispensing system **30,** i.e., the double walled-tube configuration, into bottom **26** of through-hole **24** of nose guard **20,** wherein mesh disc **40** is assembled within first tube connector **60** and first tube connector **60** and second tube connector **70** are assembled with corrugated tubing **80** assembled with bridge connector **110,** as shown in **FIGS. 16, 17****,** **19****,** **21** and **22****,** in accordance with an embodiment of the of the present invention;
**FIG. 25** schematically illustrates a front view of assembled nose guard **20,** first tube connector **60,** mesh disc **40** (not shown), dual wall drug dispensing system **30,** i.e., a double-walled tube configuration, first tube connector **60** and second tube connector **70,** bride connector **110** and corrugated tubing **80,** in accordance with an embodiment of the of the present invention;
**FIG. 26** schematically illustrates placing seal cap **150** with seal cap plug **151** into top 25 (not shown) of through-hole **24** (not shown) over nose guard **20** of assembled nose guard **20,** first tube connector **60,** mesh disc **40** (not shown), dual wall drug dispensing system **30,** i.e., a double-walled tube configuration, first tube connector **60** and second tube connector **70,** bride connector **110** and corrugated tubing **80,** in accordance with an embodiment of the of the present invention;

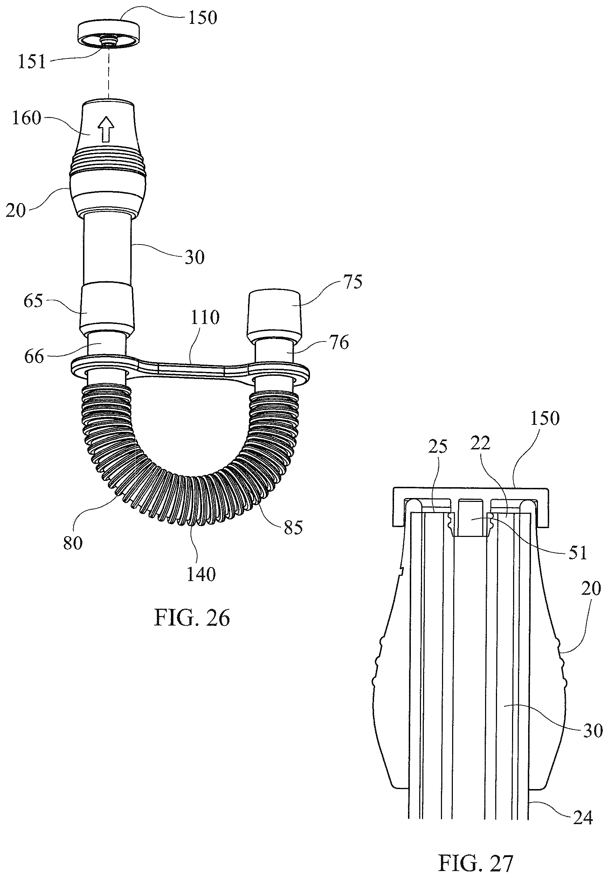

**FIG. 27** schematically illustrates an exploded cross-sectional view of nose guard **20,** wherein dual wall drug dispensing system **30,** i.e., the double walled-tube, is inserted through through-hole **24** of nose guard **20,** top end portion **31of** dual wall drug dispensing system **30,** i.e., a double-walled tube configuration, is seated against nose guard seat **22** of nose guard **20** and seal cap **150** is positioned over nose guard **20** with nose guard stem **151** is inserted through top **25** and into through-hole **24** of nose guard **20,** in accordance with an embodiment of the of the present invention;
**FIG. 28** schematically illustrates inserting bottom section **102** of one-way valve mouth piece 100 into of dual wall drug dispensing system **30,** i.e., the double walled-tube, into second top hollow collar **75** of second tube connector **70**,assembled seal cap **150,** nose guard **20,** first tube connector **60,** mesh disc **40** (not shown), dual wall drug dispensing system **30,** i.e., a double-walled tube configuration, first tube connector **60** and second tube connector **70,** bride connector **110** and corrugated tubing **80,** in accordance with an embodiment of the of the present invention;
**FIG. 29** is an exploded cross-sectional view of inner hollow chamber **33a,** having outer wall **331** and inner wall **332,** and outer hollow chamber **33b** having outer wall **333** and inner wall **334** of dual wall drug dispensing system **30,** i.e., a double-walled tube configuration, illustrating one size tube diameters, wherein, outer hollow chamber **33b** having outer wall **333** diameter of 11mm and inner wall **334** diameter of 9mm and inner hollow chamber **33a** having outer wall **331** diameter of 6mm and inner wall **332** diameter of 4mm with internal gap **36** of 1.5mm and an inner hollow chamber **33a** having an internal diameter of 4mm. Illustrations of alternative size tube diameters are: (a) outer hollow chamber **33b** having outer wall **333** diameter of 10mm and inner wall **334** diameter of 8 mm and inner hollow chamber **33a** having outer wall **331** diameter of 6mm and inner wall **332** diameter of 4mm with internal gap **36** of 1 mm and an inner hollow chamber **33a** having an internal diameter of 4mm; (b) outer hollow chamber **33b** having outer wall **333** diameter of 11mm and inner wall **334** diameter of 9mm and inner hollow chamber **33a** having outer wall **331** diameter of 5mm and inner wall **332** diameter of 3mm with internal gap **36** of 2mm and an inner hollow chamber **33a** having an internal diameter of 3mm; and (c) outer hollow chamber **33b** having outer wall **333** diameter of 13mm and inner wall **334** diameter of 11mm and inner hollow chamber **33a** having outer wall **331** diameter of 5mm and inner wall **332** diameter of 3mm with an internal gap **36** of 3mm and an inner hollow chamber **33a** having an internal diameter of 3mm, in accordance with various embodiments of the of the present invention;
**FIG. 30** is a perspective view of dual wall drug dispensing system **30,** i.e., a double-walled tube configuration, bottom end portion **32,** internal gap **36** and the two hollow chambers **33,** i.e., the inner hollow chamber **33a** and the outer hollow chamber **33b,** wherein the outer hollow chamber **33b** is divided into three separate chambers **34** by rib walls **35,** in accordance with an embodiment of the of the present invention;
**FIG. 31** is a cross-sectional view of dual wall drug dispensing system **30,** i.e., a double-walled tube configuration, as shown in **FIGS. 9, 10****,** **19** and **30****,** in accordance with an embodiment of the of the present invention;
**FIG. 32** is a cross-sectional view taken along line A of dual wall drug dispensing system 30, i.e., a double-walled tube configuration, as shown in **FIGS. 9, 10****,** **19****,** **30** and **31****,** in accordance with an embodiment of the of the present invention;
**FIG. 33** is a cross-sectional view of tube connector **60** or **70** taken along line A, as shown in **FIGS. 9, 10****,** **18****,** **23** and **31****,** in accordance with an embodiment of the of the present invention;
**FIG. 34** is a cross-sectional view of tube connector **60** or **70,** as shown in **FIGS. 9, 10****,** **18****,** **23** and **31****,** in accordance with an embodiment of the of the present invention;
**FIG. 35** is a cross-sectional view of nose guard **20** taken along line A depicting top **25** of through-hole **24,** as shown in **FIGS. 9, 10****,** **24, 25** and **27****,** in accordance with an embodiment of the of the present invention;
**FIG. 36** schematically illustrates an alternative cross-sectional view of nose guard **20,** depicting top **25** and bottom **26** of through-hole 24 and nose guard seat **22,as** shown in **FIGS. 9, 10****,** **24, 25****,** **27** and **35****,** in accordance with an embodiment of the of the present invention;
**FIG. 37** schematically illustrates an assembled view of single-directional insufflator or breath-powered nasal device **10** depicting one-way valve mouth piece **100,** first air-flow directional band **160,** first tube connector **60,** corrugated tubing **80,** bridge connector **110,** second tube connector **70,** second air-flow directional band **170,** dual wall drug dispensing system **30** and nose guard **20,** in accordance with an embodiment of the of the present invention;

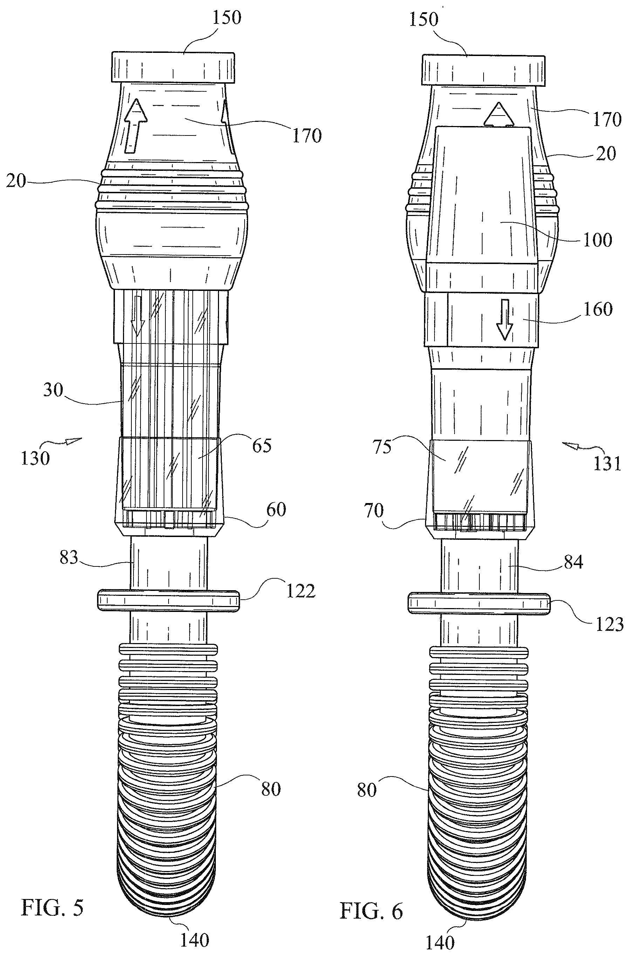

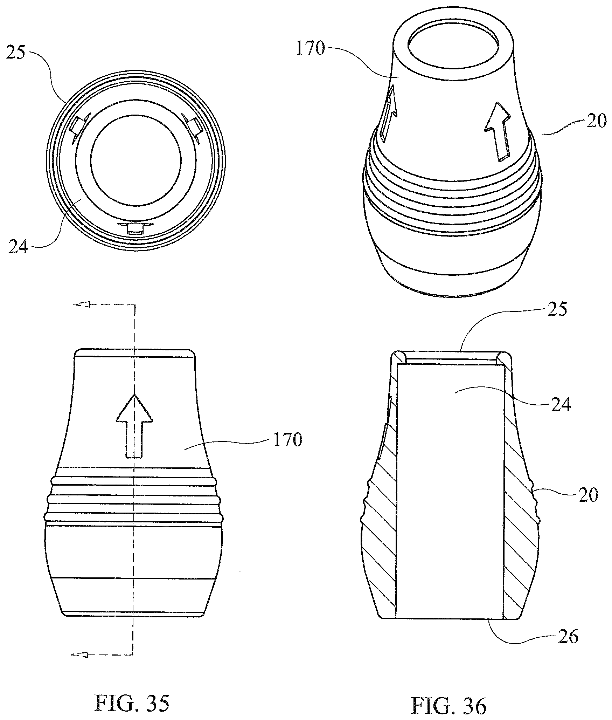

**FIG. 36** schematically illustrates an alternative one-way valve mouth piece **131** view of and assembled single-directional insufflator or breath-powered nasal device **10** nose guard, as shown in **FIG. 6****,** in accordance with an embodiment of the of the present invention;

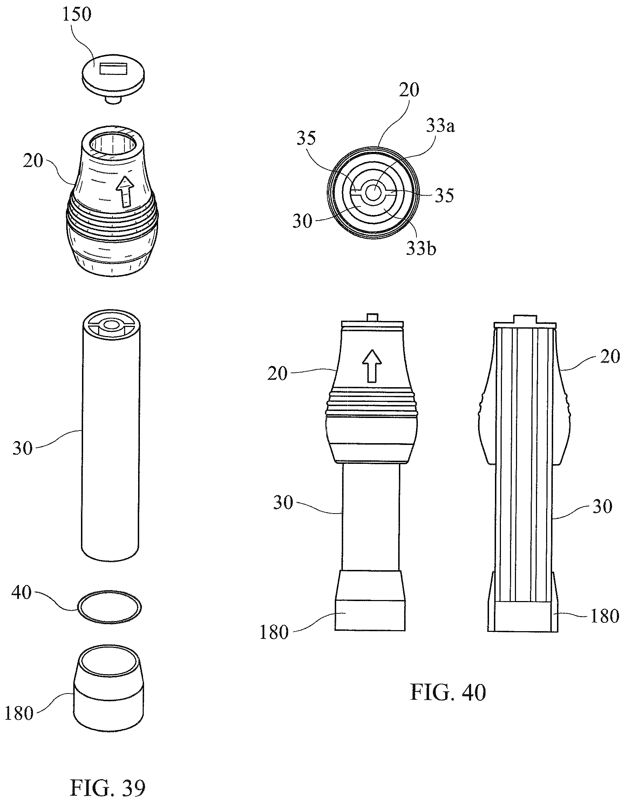

**FIG. 39** schematically illustrates an unassembled view of seal cap **150,** i.e., top plug, nose guard **20,** one-way valve dual wall drug dispensing system **30,** i.e., a double-walled tube configuration, mesh disc **40,** and alternative connector **180** for connecting one -way dual wall drug dispensing system **30,** i.e., the double walled-tube, and corrugated tubing **80,** in accordance with an embodiment of the of the present invention;
**FIG. 40** schematically illustrates an alternative cross-sectional view of seal cap **150,** i.e., top plug, nose guard **20,** and one-way valve dual wall drug dispensing system **30,** i.e., a double-walled tube configuration, assembled with alternative connector **180,** as shown in **FIG. 39****,** in accordance with an embodiment of the of the present invention;

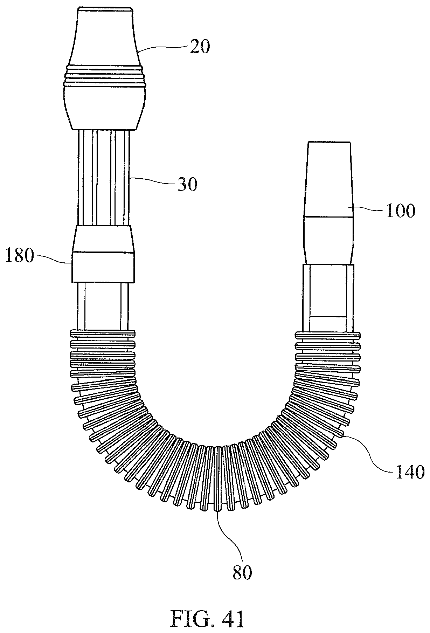

**FIG. 41** schematically illustrates an assembled front view of nose guard **20,** dual wall drug dispensing system **30,** i.e., a double-walled tube configuration, alternative connector **180,** corrugated tubing **80,** and nose guard **20,** in accordance with an embodiment of the of the present invention;

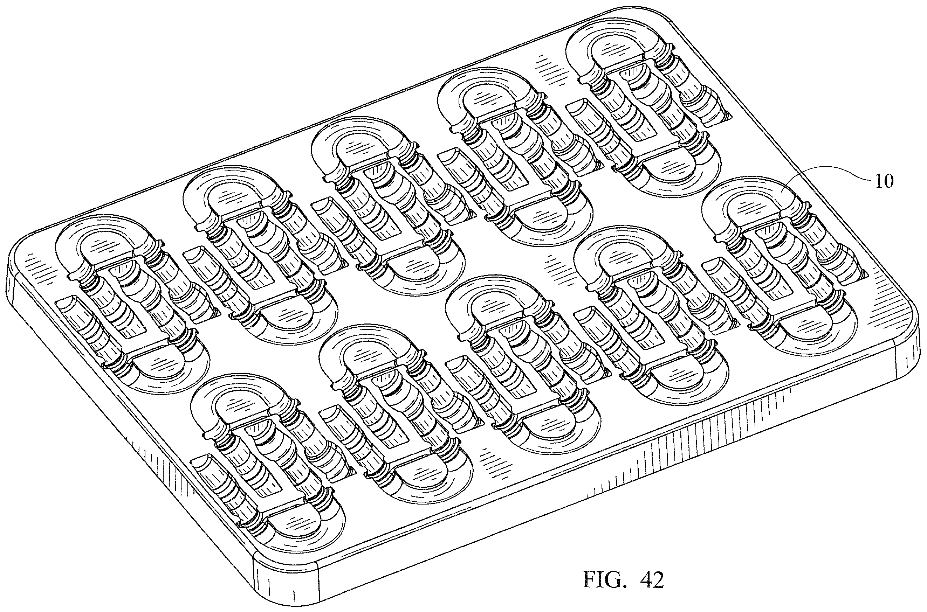

**FIG. 42** schematically illustrates a top view of a tray packaged with twenty single-directional insufflator or breath-powered nasal fully assembled devices herein, wherein the 20 such devices are positioned in two rows of five pairs of such devices, wherein each such pair shows two such devices positioned in an interlocking position with respect to one another, in accordance with an embodiment of the of the present invention;

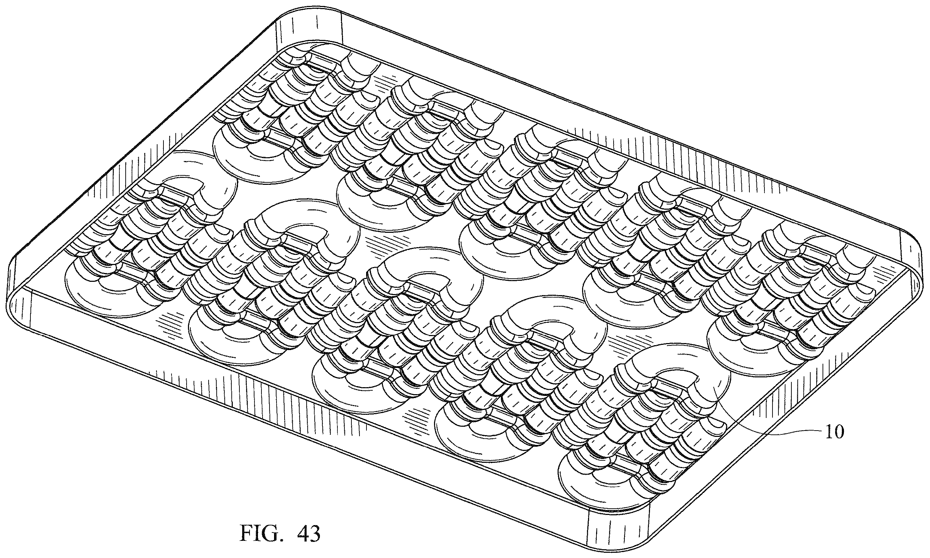

**FIG. 43** schematically illustrates a side top view of a tray packaged exposing an internal side longitudinal wall with twenty single-directional insufflator or breath-powered nasal fully assembled devices herein, wherein the 20 such devices are positioned in two rows of five pairs of such devices, wherein each such pair shows two such devices positioned in an interlocking position with respect to one another, in accordance with an embodiment of the of the present invention;

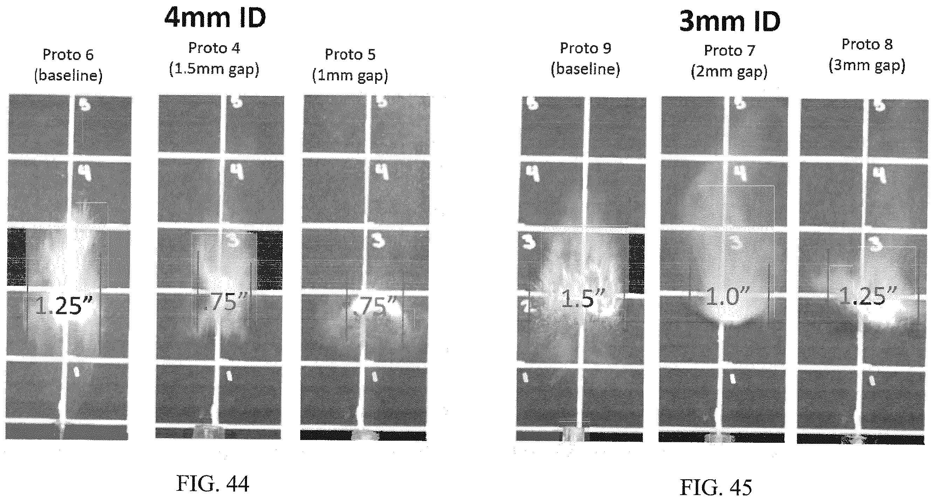

**FIG. 44** illustrates a screen shot comparing the width of a lactose powder spray between a single tube configuration and a dual wall drug dispensing system **30,** i.e., a double-walled tube configuration, of the present invention in which the internal diameter of the inner tube of the double tube is about 4mm and the gap between the inner and outer tubes of the double tube is either 1.5mm or 1mm to a single tube configuration, as compared to baseline, i.e., a single tube configuration, in accordance with an embodiment of the of the present invention;
**FIG. 45** illustrates a screen shot comparing the width of a lactose powder spray between a single tube configuration and a dual wall drug dispensing system **30,** i.e., a double-walled tube configuration, of the present invention in which the internal diameter of the inner tube of the double tube is about 3mm and the gap between the inner and outer tubes of the double tube is either 2mm or 3mm to a single tube configuration, as compared to baseline, i.e., a single tube configuration, in accordance with an embodiment of the of the present invention;

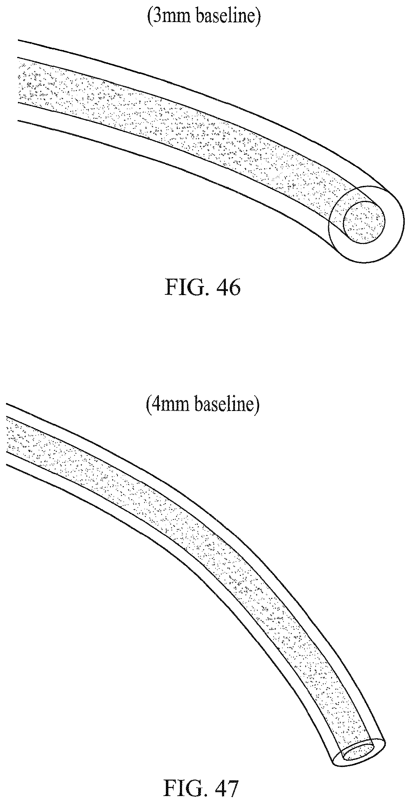

**FIG. 46** is a screen shot of a single tube configuration with a 3mm baseline following a lactose powder spray showing significant build up or accumulation of lactose powder on internal wall post-powder spray;
**FIG. 47** is a screen shot of a single tube configuration with a 4mm baseline following a lactose powder spray showing significant build up or accumulation of lactose powder on internal wall post-powder spray;

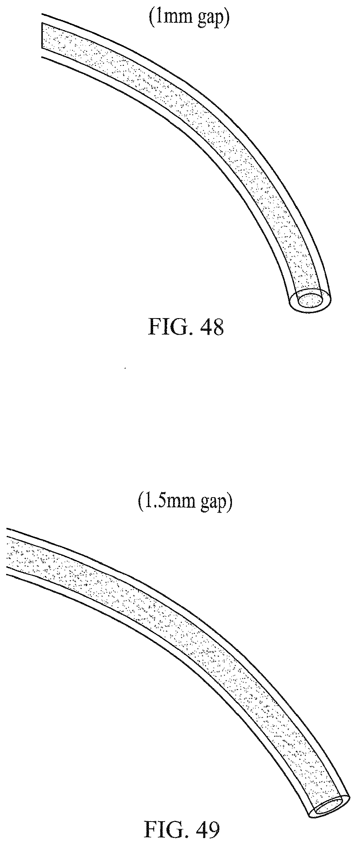

**FIG. 48** is a screen shot of a dual wall drug dispensing system **30,** i.e., a double-walled tube configuration, with a 1mm gap following a lactose powder spray showing little build up or accumulation of lactose powder on inner wall **332** of inner hollow chamber **33a** post-powder spray, as compared to the single tube configuration as shown in **FIG. 45****,** in accordance with an embodiment of the of the present invention;
**FIG. 49** is a screen shot of a dual wall drug dispensing system **30,** i.e., a double-walled tube configuration, with a 1.5 mm gap following a lactose powder spray showing little build up or accumulation of lactose powder on inner wall **332** of inner hollow chamber **33a** post-powder spray, as compared to the single tube configuration as shown in **FIG. 45****,** in accordance with an embodiment of the of the present invention;

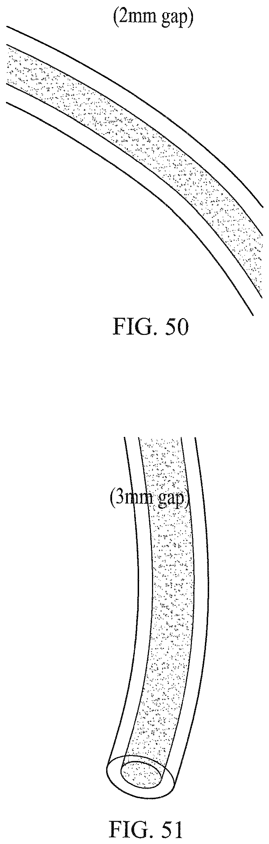

**FIG. 50** is a screen shot of a dual wall drug dispensing system **30,** i.e., a double-walled tube configuration, with a 2mm gap following a lactose powder spray showing little build up or accumulation of lactose powder on inner wall **332** of inner hollow chamber **33a** post-powder spray, as compared to the single tube configuration as shown in **FIG. 45****,** in accordance with an embodiment of the of the present invention; and
**FIG. 51** is a screen shot of a dual wall drug dispensing system **30,** i.e., a double-walled tube configuration, with a 3mm gap following a lactose powder spray showing little build up or accumulation of lactose powder on inner wall **332** of inner hollow chamber **33a** post-powder spray, as compared to the single tube configuration as shown in **FIG. 45****.**

### Description of the Invention

The present disclosure may be understood more readily by reference to the following detailed description and **FIGS.** of desired embodiments. In the following specification and the claims which follow, reference will be made to a number of terms which shall be defined to have the following meanings.

Although specific terms are used in the following description for the sake of clarity, these terms are intended to refer only to the particular structure of the embodiments selected for illustration in the drawings and are not intended to define or limit the scope of the disclosure. In the drawings and the following description below, it is to be understood that like numeric designations refer to components of like function.

The singular forms "a," "an," and "the" include plural referents unless the context clearly dictates otherwise.

The term "comprising," as used herein and in a claim, means that the named elements or steps are essential, but other elements or steps may be added, present, comprehended or included. Thus, the term "comprising", as used herein and in a claim, is synonymous with "including," "comprehending," "containing," or "characterized by," and therefore is an inclusive or open-ended claim and does not exclude additional, unrecited elements or steps. The term "comprising" should be construed to include the term "consisting of", which allows the presence of only the named components/steps.

The term "consisting essentially of," as used herein and in a claim herein, necessarily includes the recited elements or steps, but is open to unlisted elements or steps that do not materially affect the basic and material properties of the subject matter claimed, regardless of whether the subject matter is, e.g., a composition, compound, formulation, process or method.

The term "consists of," as used herein and in a claim, excludes any element or step not expressly recited and thus is a closed claim.

Numerical values should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 2 mg to 10 mg" is inclusive of the endpoints, 2 mg and 10 mg, and all the intermediate values). The endpoints of the ranges and any values disclosed herein are not limited to the precise range or value; they are sufficiently imprecise to include values approximating these ranges and/or values.

The modifier "about" used in connection with a quantity is inclusive of the stated value and has the meaning dictated by the context. When used in the context of a range, the modifier "about" should also be considered as disclosing the range defined by the absolute values of the two endpoints. For example, the range of "from about 2 to about 10" also discloses the range "from 2 to 10." The term "about" may refer to plus or minus up to 10% of the indicated number in the applicable or appropriate context. For example, "about 10%" may indicate a range of 9% to 11%, and "about 1" may mean from 0.9-1.1.

As used herein, a "composition" can be a single drug or combination drug (fixed-dose combination) combining at least the two agents, discussed *infra,* in a single dose. In another embodiment, the "composition" can include a combination of drugs or agents that are each administered or formulated separately or simultaneously. Similarly, a treatment can include administering to a patient a composition as a fixed-dose drug or agent combination, or it can include administering to a patient two or more separate agents or drugs for example, together, substantially together, concomitantly or in a predetermined sequence, frequency and time at their respective dosage strengths or amounts.

Actual dosage levels of a selective drug substance can be varied in order to achieve the effective and safe therapeutic response for a particular patient. The phrase "therapeutically effective amount" or "effective amount" means a sufficient amount of the drug substance to treat injury, diseases or disorders, at a reasonable benefit/risk ratio applicable to any medical treatment. It will be understood, however, that the total daily usage of the drug substances of the present disclosure will be decided by the attending physician or other care provider within the scope of sound medical judgment. If desired, the effective daily dose can be divided into multiple doses for purposes of administration; consequently, single dose drug substances may contain such amounts or submultiples thereof to make up the daily dose. The specific therapeutically effective dose level for any particular patient will depend upon a variety of factors, including the injury, disease or disorder being treated and the severity of the injury, disease or disorder; medical history of the patient, age, body weight, general health, sex and diet of the patient, the time of administration, route of administration, the duration of the treatment, other drugs being taken by the patient, and the like. A single administration may usually be sufficient to produce a therapeutic effect, but it is contemplated that multiple administrations will be used over a substantial period of time to assure a continued sustained response.

Accordingly, the present invention provides a single-directional insufflator or breath-powered nasal device **10** for delivering a drug substance, in powder or liquid form, to the nasal airway of a subject, comprising: a closure unit for causing the closure of the oropharyngeal velum of the subject; and a dual wall drug system **30** that provides dual airflow for delivering a vertical, somewhat tightly and narrow or confined, concentrated plume of air flow entraining a drug substance to one of the nostrils of the subject at a driving pressure which would be breath powered to drive the confined and concentrated plume deep within the nasal cavity for targeting the olfactory region, including the olfactory nerve, and the trigeminal nerve mucosa for direct diffusion into the brain and for effective treatment of nasal and/or CNS injury, disease or disorder, in particular TBI, especially concussion, within minutes of nasal application, wherein a single-directional insufflator or breath-powered nasal device **10** of the present invention comprises a nose guard **20** which includes an outlet through which the breath powered air flow is delivered to the one nostril for providing a fluid tight seal between the nose guard **20** and the one nostril.

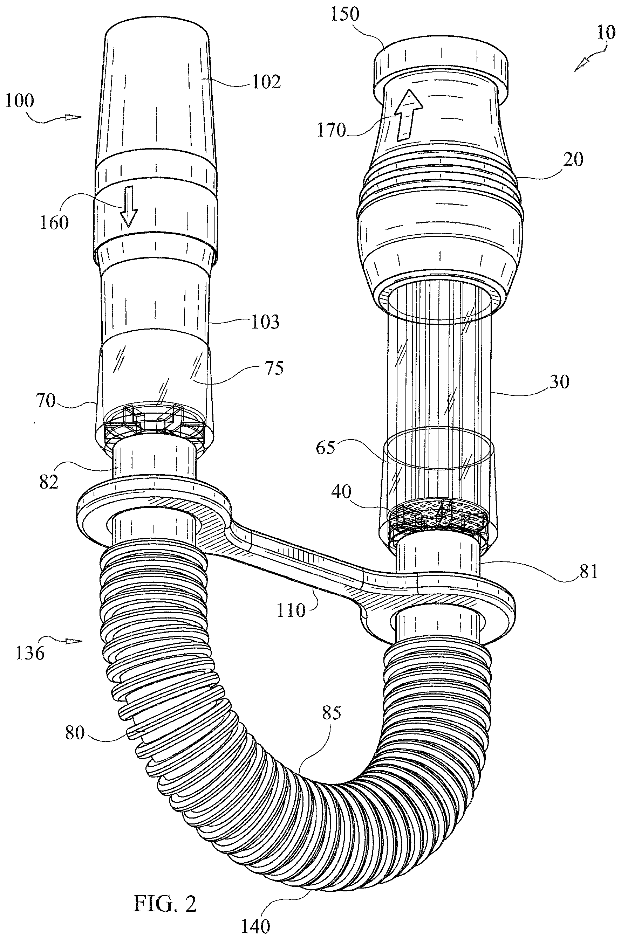

More specifically, single-directional insufflator or breath-powered nasal device **10** of the present invention comprises a nose guard **20,** a one-way valve mouth piece **100,** corrugated tubing **80** and means for propelling a dual airflow, e.g., dual wall drug dispensing system **30,** i.e., a double-walled tube configuration, for propelling a first airflow carrying the powdered drug substance and propelling a second airflow for surrounding the first airflow for propelling highly concentrated powdered drug substance deep within the nasal cavity, as shown, for example, in **FIGS.** 1 and **2****.**

More specifically speaking, the single-directional insufflator or breath-powered nasal device **10** of the present invention is comprised of nose guard **20,** dual wall drug dispensing system **30,** i.e., a double-walled tube configuration, mesh disc **40,** first tube connector **60,** corrugated tubing **80,** second tube connector **70,** and a one-way valve mouth piece **100,** as shown in **FIGS. 1-2** and **7-10.**

The nose guard **20** is tapered and configured with a through-hole **24** and an internal collar **26** against which the top portion **31** of the dual wall drug dispensing system **30** rest when the dual wall drug dispensing system **30,** i.e., a double-walled tube configuration, is fully inserted into aperture **24** of nose guard **20,** as shown in **FIGS. 9-10** and **23-24.** Nose guard **20** can be manufactured out of any material that is suitable for nose guard's **20** intended purposes. Seal cap **150,** i.e., top cover, as shown in **FIGS. 1****, 9-10**, **13-14** and **26-28,** may be inserted into the top **25** of aperture **24** to seal nose guard **20.**

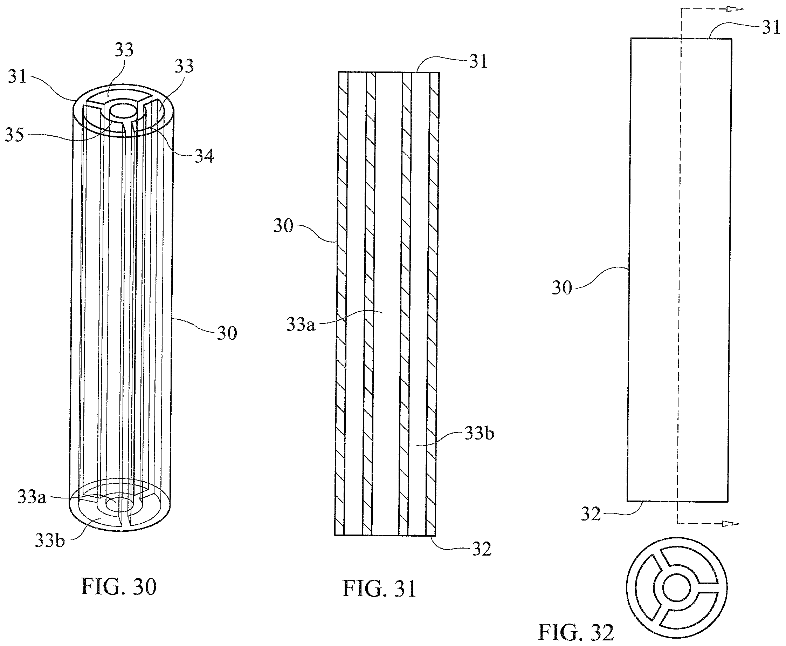

The dual wall drug dispensing system **30,** is a novel and unique system for delivering one or more drug substances into the nasal cavity and an important aspect of the present invention. The dual wall drug dispensing system **30,** i.e., a double-walled tube configuration, is preferably extruded and comprises a top end portion **31** and the bottom end portion **32** and two hollow chambers **33,** an inner hollow chamber **33a** and an outer hollow chamber **33b.** The hollow inner chamber **33a** holds drug substance **200** (not shown). The hollow outer chamber **33b,** which surrounds the hollow inner chamber **33a,** is divided into two, three, four or more separate chambers **34** by rib walls **35.** Preferably, hollow outer chamber **33b** is divided into three separate chambers **34.** See, for example, **FIGS. 30-32****.**

Bridge connector **110** connects one-way valve mouth piece side **131** comprising the one-way valve mouth piece **100** and second tube connector **70** to nose guard side **130** comprising the nose guard **20,** dual wall drug dispensing system **30,** i.e., a double-walled tube configuration, mesh disc **40,** first tube connector **60** and corrugated tubing **80** to maintain the breath-powered insufflator or breath-powered nasal device **10** of the present invention in a constant "U-shape" configuration **140.** Bridge connector **120** comprises a bridge **121** sandwiched between two hollow rings **122** and **123,** respectively, for sliding over first hollow cuff **81** and second hollow cuff **82,** respectively of convoluted middle section with corrugations **80,** as shown in **FIGS. 1-4** **and** **7-12****,** **16-17-19****,** **21-22****,** **24-26** and **28.**

Alternatively, the bridge connector **120** may be designed with a perforation or score line **124** or any other configuration that allows bridge connector **120** to be broken in two pieces in the event sides **130** and **131** of the device need to be straightened for use by a second person who is assisting, e.g., a concussed person who cannot self-administer the necessary blow force to provide sufficient airflow to effectively propel the drug substance from the single-directional insufflator or breath-powered nasal device **10** of the present invention to effectively treat the concussion with the drug substance. See **FIGS. 13** and **14****.**

The dual wall drug dispensing system **30,** i.e., a double-walled tube configuration, is a novel and unique system for delivering one or more drug substances into the nasal cavity and an important aspect of the present invention. The dual wall drug dispensing system **30,** i.e., a double-walled tube configuration, comprises a top end portion **31** and the bottom end portion **32** and two hollow chambers **33,** an inner hollow chamber **33a** and an outer hollow chamber **33b.** The hollow inner chamber **33a** holds drug substance **200.** The hollow outer chamber **33b,** which surrounds the hollow inner chamber **33a,** is divided into two, three, four or more separate chambers **34** by rib walls **35.** Preferably, hollow outer chamber **33b** is divided into three separate chambers **34.** See, for example, **FIGS. 29-32****.**

First and second tube connectors **60** and **70** are each provided with first top hollow collar **65** and second top hollow collar **75,** respectively, for insertion into first hollow cuff **81** and second hollow cuff **82,** respectively, of corrugated tubing **80.** However, before first top hollow collar **65** and second top hollow collar **75** are inserted into respective first hollow cuff **81** and second hollow cuff **82of** corrugated tubbing **80,** bridge connector **110** the first hollow ring **122** and second hollow ring **123** of bridge connector **110** is slid over first hollow cuff **81** and second hollow cuff **82,** respectively, to create a u-shape configuration **140.** See **FIGS. 16-17****.**

As described herein before, to load the drug substance **200,** when in a powdered format, especially a spray-dried format, into the novel single-directional insufflator or breath-powered nasal device **10** of the present invention, a sterilized funnel (not shown) is used to load the powdered drug substance **200** specifically within the inner hollow chamber **33a** of the dual wall drug dispensing system **30,** i.e., a double-walled tube configuration. The mesh disc **40,** which is positioned at bottom end portion **32** of the inner hollow chamber **33a,** at the top **66** of the first tube connector **60,** is what the loaded powdered drug substance **200** first encounters and against which it rest once fully loaded. Because the pore size of the mesh disc **40** is smaller than the particle size of the powdered drug substance, the powdered drug substance cannot fall through the mesh disc **40.** The pores throughout the mesh disc **40,** however, permit the blown airflow to penetrate up and through the mesh disc **40** to carry and propel the powdered drug substance **200** from the inner hollow chamber **33a** out of nose guard **20** and into the nasal cavity.

Mesh disc **40,** as shown for example in **FIG. 1****,** can be manufactured from a multitude of different material options. For example, mesh disc **40** can be manufactured with a PET material. Different materials and mesh size could be used for different applications. By way of example, it may be possible to use liquid with use of a PTFE hydrophobic membrane to contain the dose (depending on chemistry of course). One example of a material contemplated by the present invention for mesh disc **40** is SEFAR MEDIFAB 07-135/40 158 cm, Item Number: 3A07-0135-158-00. One example of a sized mesh disc **40** manufactured with the SEFAR MEDIFAB 07-135/40 158 cm, Item Number: 3A07-0135-158-00 material, that is contemplated for use in accordance with the present, is a mesh disc **40** having a diameter of about 0.411, a thickness of from about 0.004 inches to about 0.012 inches or more, and a surface roughness of about 125µin.

Preferably, the particle size distribution of the powdered, spray-dried, drug substance **200** is principally in the range of about 1µm to about 10µm. In one embodiment, the powdered, spray-dried, drug substance contains a medicament, particularly for the treatment of a nasal condition. Notwithstanding the preferred particle size, the present invention contemplates particle size distribution of a powdered, spray-dried, drug substance to include larger particles, or a smaller fraction of larger particles, typically in the range of about 10µm to about 30µm, and preferably in the range of about 20µm to about 30µm.

By nesting the inner hollow chamber **33a** with powdered, spray-dried, drug substance 200 (not shown) inside the unobstructed larger outer hollow chamber **33b,** as shown **FIGS. 1-2****,** **7-**and **30,** dual wall drug dispensing system **30,** i.e., a double walled-tubing configuration, will allow for unmedicated airflow to engage the sidewall of the nasal cavity while allowing the airflow carrying the powdered, spray-dried, drug substance **200,** which flows in the center of the unmedicated airflow, to reach its targeted destination deep inside the nasal cavity with minimal drug substance **200** loss.

This benefit is illustrated in **FIGS. 48-51****,** where there is little dry powder build up on the inner walls **332** of inner hollow chambers **33a** of dual wall drug dispensing systems **30,** i.e., a double walled-tubing configuration, post-powder spray through inner hollow chambers **33a,** as compared to the significant dry powder build up on the inner walls of single tube constructions with 3mm or 4mm baseline, **FIG. 46****,** and the significant dry powder build up on the inner wall of single tube construction with mm baseline, **FIG. 47****,and** making both tubes almost opaque, post-powder spray through the single tube construction. In contrast, and as shown in **FIGS. 48-51****,** the inner walls **332** of inner hollow chambers **33a** of dual wall drug dispensing systems **30,** i.e., a double walled-tubing configuration, with 1mm gap, 1.5mm gap, 2mm gap and 3mm gap, respectively, have a light dusting, with very little powder build up, post-powder spray through inner hollow chamber **33a** indicating that more dry powder reaches the targeted destination.

One-way valve mouth piece **100,** as shown in **FIGS. 1-2****, 9-10** and **28,** comprises there through conduit **101** with top section **102** and bottom section **103,** respectively. Within bottom section **103** sits a one-way valve (**not shown**) for directing blown air into corrugated tube **80** and preventing reverse airflow. One-way valve mouth piece **100** is designed for insertion into second collar **75** of second tube connector **70** against which bottom section **103** sits when one-way valve mouth piece **100** is fully press-fit into second collar 75 of second tube connector 70. An example of a one-way valve mouth piece **100** contemplated by the present invention is Crosstex Safe-Flo^{™} Adapter, M1020ADAP one-way valve mouth piece manufactured by Crosstex International. The Safe-Flo^{™} Adapter is designed with a unique, built-in one-way valve to provide a physical barrier that prevents backflow.

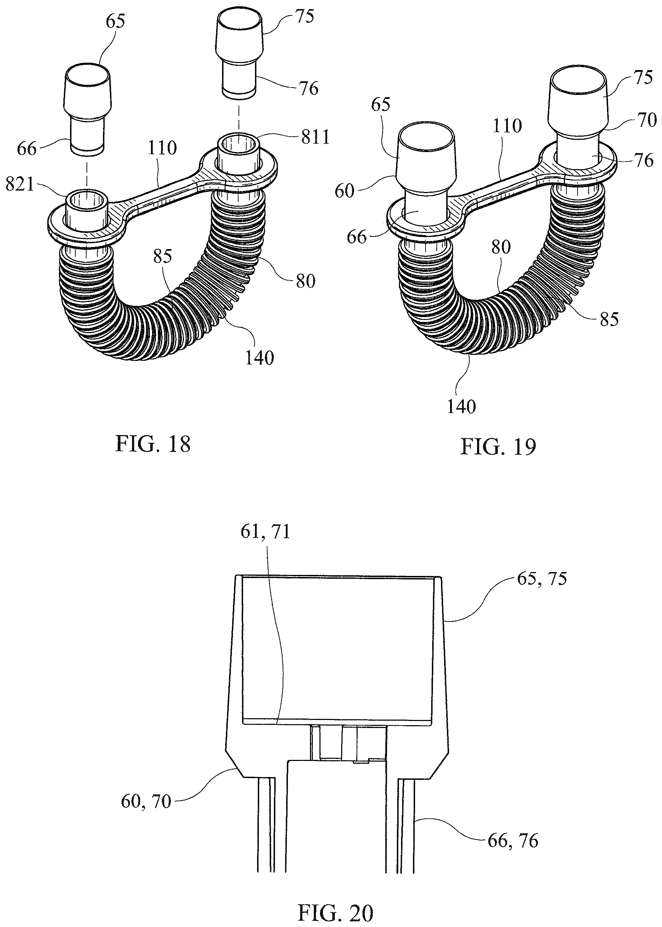

Corrugated tubing **80** is arched to a degree that allows nose guard side **130** and **131** to form a U-shaped configuration **140,** as shown in **FIGS. 1-2****, 7-8****,** **13****,** **11****,** **13** and **37.** Corrugated middle section **85** of corrugated tubing **80** has first and second top ends **811** and **821** for respectively receiving first bottom hollow neck **66** of first tube connector **60** and second bottom hollow neck **76** of second tube connector **70** to further the assembly of the single-directional insufflator or breath-powered nasal device **10** of the present invention, as shown in **FIGS. 15** **and** **18****.**

**FIG. 1** is a front assembled elevational angled front view **135** of a single-directional insufflator or breath-powered nasal device **10.**

**FIG. 2** is a rear assembled elevational angled back view **136** of a single-directional insufflator or breath-powered nasal device **10.**

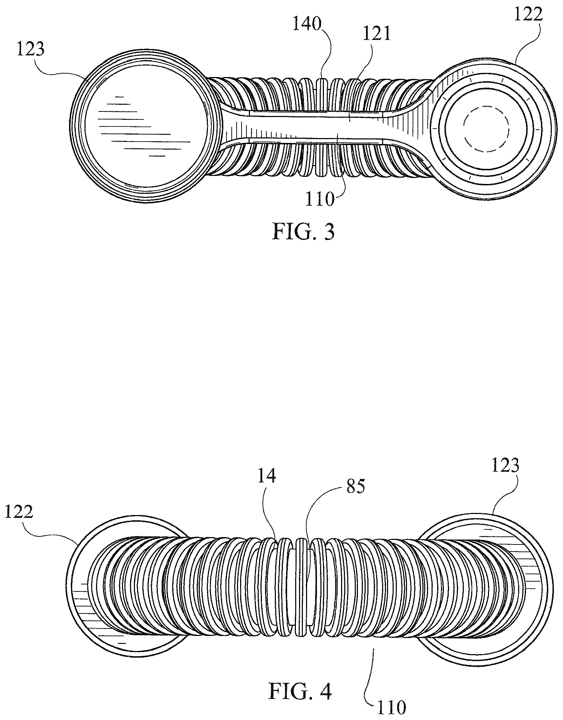

**FIG. 3** is a top view **850** of a corrugated tube **80** showing corrugated middle section **85** and bridge connector **110.**

**FIG. 4** is a bottom view **860** of a corrugated tube **80** showing corrugated middle section **85.**

**FIG. 5** **is** an assembled elevational view of nose guard side **130** of the single-directional insufflator or breath-powered nasal device **10.**

**FIG. 6** is an assembled elevational view of the one-way valve mouth piece side **131** of the single-directional insufflator or breath-powered nasal device **10.**

**FIG. 7** is a right mouth piece assembled elevational view of a single-directional insufflator or breath-powered nasal device 10.

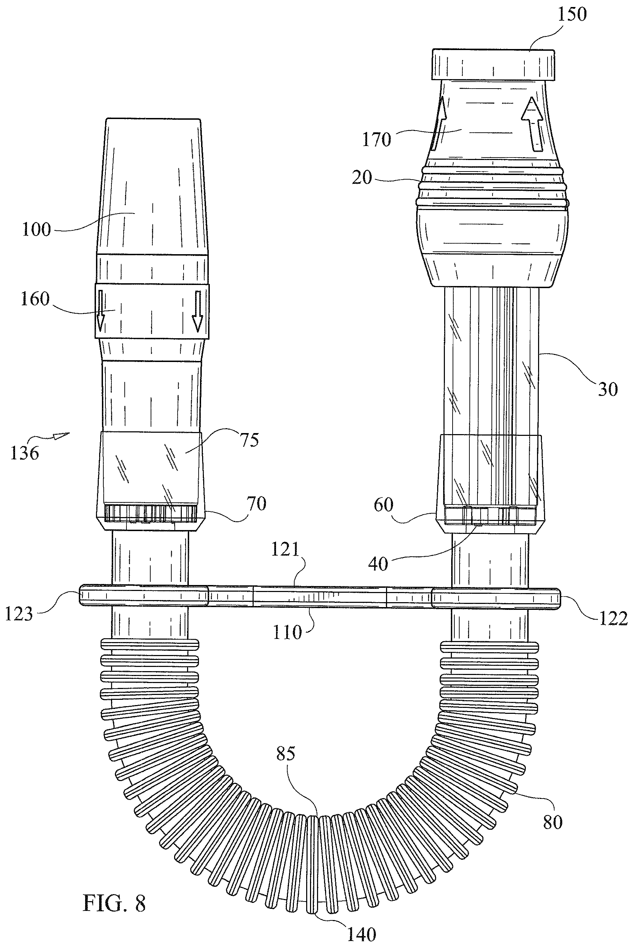

**FIG. 8** is a left mouth piece assembled elevational view of a single-directional insufflator or breath-powered nasal device **10.**

**FIG. 9** is a right mouth piece front unassembled view of a single-directional insufflator or breath-powered nasal device **10.**

**FIG. 10** is a left mouth piece front unassembled view of a single-directional insufflator or breath-powered nasal device **10.**

**FIG. 11** illustrates single-directional insufflator or breath-powered nasal device **10,** as shown in **FIGS. 1-2** and **4-8,** inserted into a nasal cavity of a subject for operation.

**FIG. 12** illustrates nose guard **20** of a single-directional insufflator or breath-powered nasal device **10,** as shown in **FIGS. 13** and **14****,** that is inserted into a nasal cavity of a first subject to be treated with a drug substance 200 (not shown) and one-way valve mouth piece **100,** as shown in **FIGS. 13** and **14****,** inserted between the lips of a second subject for operation of the alternative embodiment by the second subject as shown in **FIGS. 13** and **14****.**

**FIG. 13** is a front, right mouth piece, assembled elevational angled view of a single-directional insufflator or breath-powered nasal device 10, wherein the bridge connector **110** is designed with a perforated or score line **142** for breaking bridge connector **110** in two.

**FIG. 14** is a front, right mouth piece, assembled elevational angled view of a single-directional insufflator or breath-powered nasal device **10,** wherein the bridge connector **110** is broken in two pieces at the perforated or score line **142** for breaking bridge connector **110** into two separate pieces, as shown in **FIG. 13****.**

**FIG. 15** illustrates assembling bridge connector **110** to corrugated tubing **80.**

**FIG. 16** illustrates a perspective view of bridge connector **110** and corrugated tubing **80** in an assembled configuration.

**FIG. 17** illustrates a front view of bridge connector **110** and corrugated tubing **80** in an assembled configuration.

**FIG. 18** illustrates assembling first tube connector **60** and second tube connector 70 to corrugated tubing **80** assembled with bridge connector **110,** as shown in **FIGS. 16** and **17****.**

**FIG. 19** illustrates a perspective view of first tube connector **60** and second tube connector **70** assembled with corrugated tubing **80** assembled with bridge connector **110,** as shown in **FIGS. 16** and **17****.**

**FIG. 20** illustrates a cross-sectional view of tube connectors **60** and **70.**

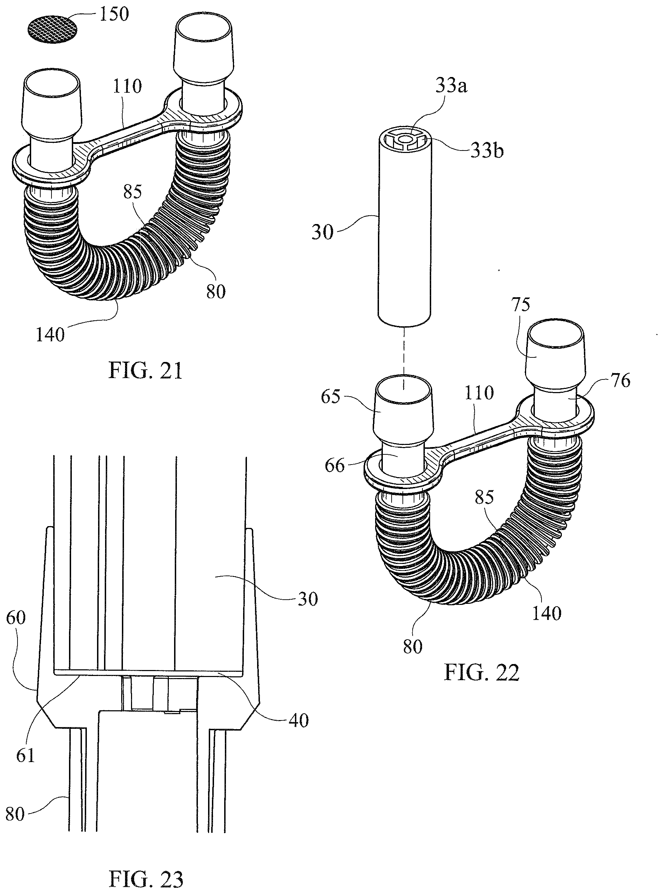

**FIG. 21** illustrates inserting a mesh disc **40** into first tube connector **60,** wherein first tube connector **60** and second tube connector **70** are assembled with corrugated tubing **80** assembled with bridge connector **110,** as shown in **FIGS. 16** and **17****.**

**FIG. 22** illustrates inserting a dual wall drug dispensing system **30,** i.e., a double-walled tube configuration, into first tube connector **60,** wherein mesh disc **40** is inserted into first tube connector **60** and first tube connector **60** and second tube connector **70** are assembled with corrugated tubing **80** assembled with bridge connector **110,** as shown in **FIGS. 16, 17****,** **19** and **21****.**

**FIG. 23** illustrates an exploded cross-sectional view of assembled first tube connector **60,** wherein mesh disc **40** and dual wall drug dispensing system **30,** i.e., a double-walled tube configuration, are assembled within first tube connector **60** and mesh disc **40** is sitting on first seat **61** of first tube connector **60** and first top hollow collar **65** of first tube connector **60** is press-fit into first hollow cuff **81** of corrugated tubing **80.**

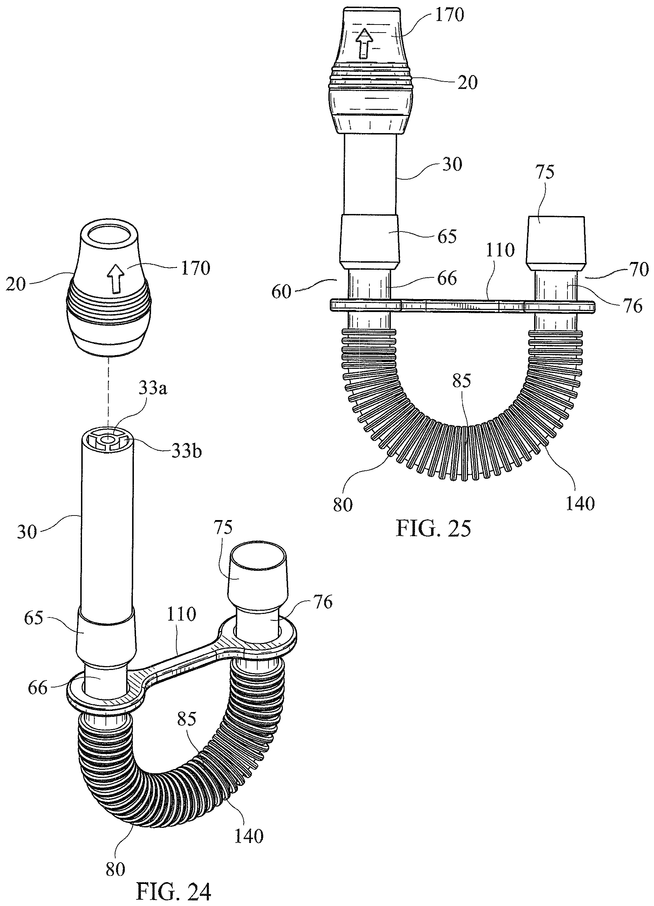

**FIG. 24** illustrates inserting top end portion **31** of dual wall drug dispensing system **30,** i.e., the double walled-tube configuration, into bottom **26** of through-hole **24** of nose guard **20,** wherein mesh disc **40** is assembled within first tube connector **60** and first tube connector **60** and second tube connector **70** are assembled with corrugated tubing **80** assembled with bridge connector **110,** as shown in **FIGS. 16, 17****,** **19****,** **21** and **22****.**

**FIG. 25** illustrates a front view of assembled nose guard **20,** first tube connector **60,** mesh disc **40** (not shown), dual wall drug dispensing system **30,** i.e., a double-walled tube configuration, first tube connector **60** and second tube connector **70,** bride connector **110** and corrugated tubing **80.**

**FIG. 26** illustrates placing seal cap **150** with seal cap plug **151** into top 25 (not shown) of through-hole **24** (not shown) over nose guard **20** of assembled nose guard **20,** first tube connector **60,** mesh disc **40** (not shown), dual wall drug dispensing system **30,** i.e., a double-walled tube configuration, first tube connector **60** and second tube connector **70,** bride connector **110** and corrugated tubing **80.**

**FIG. 27** illustrates an exploded cross-sectional view of nose guard **20,** wherein dual wall drug dispensing system **30,** i.e., the double walled-tube, is inserted through through-hole **24** of nose guard **20,** top end portion **31of** dual wall drug dispensing system **30,** i.e., a double-walled tube configuration, is seated against nose guard seat **22** of nose guard **20** and seal cap **150** is positioned over nose guard **20** with nose guard stem **151** is inserted through top **25** and into through-hole **24** of nose guard **20.**

**FIG. 28** illustrates inserting bottom section **102** of one-way valve mouth piece 100 into of dual wall drug dispensing system **30,** i.e., the double walled-tube, into second top hollow collar **75** of second tube connector **70,**assembled seal cap **150,** nose guard **20,** first tube connector **60,** mesh disc **40** (not shown), dual wall drug dispensing system **30,** i.e., a double-walled tube configuration, first tube connector **60** and second tube connector **70,** bride connector **110** and corrugated tubing **80.**

**FIG. 29** is an exploded cross-sectional view of inner hollow chamber **33a,** having outer wall **331** and inner wall **332,** and outer hollow chamber **33b** having outer wall **333** and inner wall **334** of dual wall drug dispensing system **30,** i.e., a double-walled tube configuration, illustrating one size tube diameters, wherein, outer hollow chamber **33b** having outer wall **333** diameter of 11mm and inner wall **334** diameter of 9mm and inner hollow chamber **33a** having outer wall **331** diameter of 6mm and inner wall **332** diameter of 4mm with internal gap **36** of 1.5mm and an inner hollow chamber **33a** having an internal diameter of 4mm. Illustrations of alternative size tube diameters are: (a) outer hollow chamber **33b** having outer wall **333** diameter of 10mm and inner wall **334** diameter of 8 mm and inner hollow chamber **33a** having outer wall **331** diameter of 6mm and inner wall **332** diameter of 4mm with internal gap **36** of 1 mm and an inner hollow chamber **33a** having an internal diameter of 4mm; (b) outer hollow chamber **33b** having outer wall **333** diameter of 11mm and inner wall **334** diameter of 9mm and inner hollow chamber **33a** having outer wall **331** diameter of 5mm and inner wall **332** diameter of 3mm with internal gap **36** of 2mm and an inner hollow chamber **33a** having an internal diameter of 3mm; and (c) outer hollow chamber **33b** having outer wall **333** diameter of 13mm and inner wall **334** diameter of 11mm and inner hollow chamber **33a** having outer wall **331** diameter of 5mm and inner wall **332** diameter of 3mm with an internal gap **36** of 3mm and an inner hollow chamber **33a** having an internal diameter of 3mm.

**FIG. 30** is a view of dual wall drug dispensing system **30,** i.e., a double-walled tube configuration, bottom end portion **32,** internal gap **36** and the two hollow chambers **33,** i.e., the inner hollow chamber **33a** and the outer hollow chamber **33b,** wherein the outer hollow chamber **33b** is divided into three separate chambers **34** by rib walls **35.**

**FIG. 31** is a cross-sectional view of dual wall drug dispensing system **30,** i.e., a double-walled tube configuration, as shown in **FIGS. 9, 10****,** **19** and **30****.**

**FIG. 32** is a cross-sectional view taken along line A of dual wall drug dispensing system **30,** i.e., a double-walled tube configuration, as shown in **FIGS. 9, 10****,** **19****,** **30** and **31****.**

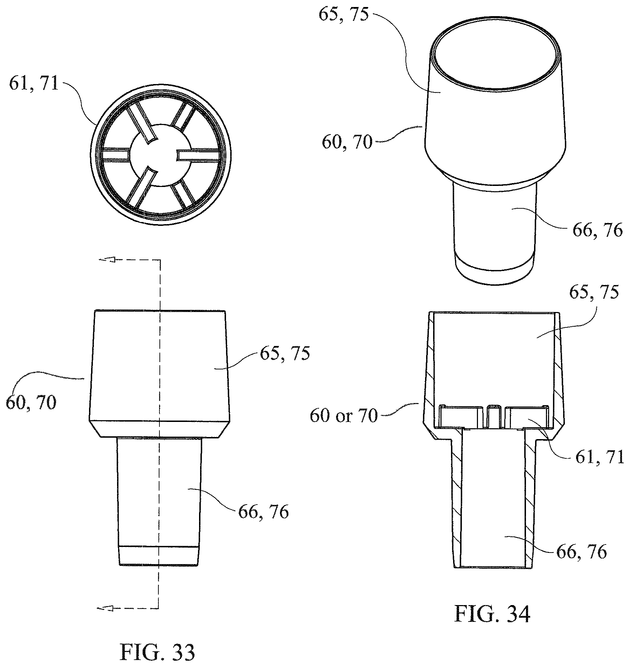

**FIG. 33** is a cross-sectional view of tube connector **60** or **70** taken along line A, as shown in **FIGS. 9, 10****,** **18****,** **23** and **31****.**

**FIG. 34** is a cross-sectional view of tube connector **60** or **70,** as shown in **FIGS. 9, 10****,** **18****,** **23** and **31****.**

**FIG. 35** **is** a cross-sectional view of nose guard **20** taken along line A depicting top **25** of through-hole **24,** as shown in **FIGS. 9, 10****,** **24, 25** and **27****.**

**FIG. 36** illustrates an alternative cross-sectional view of nose guard **20,** depicting top **25** and bottom 26 of through-hole 24 and nose guard seat **22,**as shown in **FIGS. 9, 10****,** **24, 25****,** **27** and **35****.**

**FIG. 37** illustrates an assembled view of single-directional insufflator or breath-powered nasal device **10** depicting one-way valve mouth piece **100,** first air-flow directional band **160,** first tube connector **60,** corrugated tubing **80,** bridge connector **110,** second tube connector **70,** second air-flow directional band **170,** dual wall drug dispensing system **30** and nose guard **20.**

**FIG. 36** illustrates an alternative one-way valve mouth piece **131** view of and assembled single-directional insufflator or breath-powered nasal device **10** nose guard, as shown in **FIG. 6****.**

**FIG. 39** illustrates an unassembled view of seal cap **150,** i.e., top plug, nose guard **20,** one-way valve dual wall drug dispensing system **30,** i.e., a double-walled tube configuration, mesh disc **40,** and alternative connector **180** for connecting one -way dual wall drug dispensing system **30,** i.e., the double walled-tube, and corrugated tubing **80.**

**FIG. 40** illustrates an alternative cross-sectional view of seal cap **150,** i.e., top plug, nose guard 20, and one-way valve dual wall drug dispensing system **30,** i.e., a double-walled tube configuration, assembled with alternative connector **180,** as shown in **FIG. 39****;**

**FIG. 41** illustrates an assembled front view of nose guard **20,** dual wall drug dispensing system **30,** i.e., a double-walled tube configuration, alternative connector **180,** corrugated tubing **80,** and nose guard **20.**

**FIG. 42** illustrates a top view of a tray packaged with twenty single-directional insufflator or breath-powered nasal fully assembled devices herein, wherein the 20 such devices **10** are positioned in two rows of five pairs of such devices, wherein each such pair shows two such devices positioned in an interlocking position with respect to one another.

**FIG. 43** illustrates a side top view of a tray packaged exposing an internal side longitudinal wall with twenty single-directional insufflator or breath-powered nasal fully assembled devices herein, wherein the 20 such devices **10** are positioned in two rows of five pairs of such devices, wherein each such pair shows two such devices positioned in an interlocking position with respect to one another.

**FIG. 44** illustrates a screen shot comparing the width of a lactose powder spray between a single tube configuration and a dual wall drug dispensing system **30,** i.e., a double-walled tube configuration, of the present invention in which the internal diameter of the inner tube of the double tube is about 4mm and the gap between the inner and outer tubes of the double tube is either 1.5mm or 1mm to a single tube configuration.

**FIG. 45** illustrates a screen shot comparing the width of a lactose powder spray between a single tube configuration and a dual wall drug dispensing system **30,** i.e., a double-walled tube configuration, of the present invention in which the internal diameter of the inner tube of the double tube is about 3mm and the gap between the inner and outer tubes of the double tube is either 2mm or 3mm to a single tube configuration.

As shown in **FIGS. 44 and 45****,** when comparing the width of the powder spray at the same height, the spray width of dual wall drug dispensing system **30,** i.e., a double-walled tube configuration, with internal diameters of 4mm and 3mm, respectively, of inner hollow chambers **33a** stays more centered that that of a baseline single tubular configuration. In other words, the baseline single tubular configuration spread out wider and sooner than dual wall drug dispensing system **30,** i.e., a double-walled tube configuration. These results demonstrate that dual wall drug dispensing system **30,** i.e., a double-walled tube configuration, would allow drug substance 200 (not shown) to possibly reach deeper into a nasal cavity, rather than collecting on the walls of the nose. In addition, because it is believed that the 3mm ID devices **30** may disperse spray powder at lower heights and reach lower maximum heights than the 4mm ID devices **30** of **FIGS. 44-45****,** dual wall drug dispensing system **30,** i.e., a double-walled tube configuration of the present invention that are configured with inner hollow chambers **33a** that have internal diameters of about 4mm ID or greater are preferred.

**FIG. 46** illustrates a screen shot of a single tube configuration with a 3 mm baseline following a lactose powder spray showing significant build up or accumulation of lactose powder on internal wall post-powder spray, as compared to the single tube configuration as shown in FIG. **45****.**

**FIG. 47** illustrates a screen shot of a single tube configuration with a 4 mm baseline following a lactose powder spray showing significant build up or accumulation of lactose powder on internal wall post-powder spray, as compared to the single tube configuration as shown in FIG. 45.

**FIG. 48** illustrates a screen shot of a dual wall drug dispensing system **30,** i.e., a double-walled tube configuration, with a 1mm gap following a lactose powder spray showing little build up or accumulation of lactose powder on inner wall **332** of inner hollow chamber **33a** post-powder spray, as compared to the single tube configuration as shown in FIG. **45****.**

**FIG. 49** illustrates a screen shot of a dual wall drug dispensing system **30,** i.e., a double-walled tube configuration, with a 1.5mm gap following a lactose powder spray showing little build up or accumulation of lactose powder on inner wall **332** of inner hollow chamber **33a** post-powder spray, as compared to the single tube configuration as shown in FIG. **45****.**

**FIG. 50** illustrates a screen shot of a dual wall drug dispensing system **30,** i.e., a double-walled tube configuration, with a 2mm gap following a lactose powder spray showing little build up or accumulation of lactose powder on inner wall **332** of inner hollow chamber **33a** post-powder spray, as compared to the single tube configuration as shown in FIG. **45****.**

**FIG. 51** illustrates a screen shot of a dual wall drug dispensing system **30,** i.e., a double-walled tube configuration, with a 3mm gap following a lactose powder spray showing little build up or accumulation of lactose powder on inner wall **332** of inner hollow chamber **33a** post-powder spray, as compared to the single tube configuration as shown in FIG. **45****.**

It should be understood that each part connection described herein is an airtight connection to ensure that blown airflow does not escape and travels in a harmonius single direction.

It should be further understood that top **25** of through-hole **24** of nose guard **20** and top **102** of aperture **101** of one-way valve mouth piece **100** may each be sealed with any effective sealing means to seal nose guard **20** and one-way valve mouth piece **100** from external air or other contamination and moisture, such as a top plug **150** designed to seal top **25** of through-hole **24** of nose guard **20** and top section **102** of conduit **101** when sized to fit accordingly. The present invention also contemplates other suitable sealing means that can serve as alternatives to top plug **150,** such as seals using induction foil or shrink wrap, so long as the objectives of the present invention are accomplished and not defeated.

In a preferred embodiment, where the substance is in solid form, such as a powder, the present invention contemplates use of a filter or desiccant if high humidity represents a problem for administration of the solid.

The drug substance **200** can be a single compound or a mixture of compounds, which compounds can be in any suitable form, such as a powder form, a solution, a suspension, a gel or the like.

The drug substance **200** can be any suitable substance for delivery to a human or in some cases an animal. The drug substance **200** may be for delivery for action deep within the nasal cavity for direction diffusion into the brain and/or for topical or systemic action that may require nasal administration of a drug substance **200** for treatment. Thus, a drug substance **200** may be for systemic delivery for action in a region remote from the nasal airway.

Preferably, the drug substance **200** is for delivery to the olfactory region and the trigeminal nerve mucosa for rapid diffusion into the brain for the treatment of nasal and/or CNS injury, disease or disorder, especially brain injury, such as TBI (mild, moderate and/or severe), including concussion.

The drug substance **200** may have a beneficial medical effect, which can include a diagnostic effect, a therapeutic effect, a curative effect, a systemic effect and/or a prophylactic effect, etc. Preferably, the drug substance **200** has a therapeutic or curative effect.

Preferably, the drug substance **200** is a pharmaceutical. The pharmaceutical can be admixed with any suitable carrier, diluent, excipient or adjuvant to form a pharmaceutical composition.

There are many nasal conditions which require treatment. One such condition is brain trauma known, *i.e.,* mild, moderate and severe TBI, including concussion. Another such condition is nasal inflammation, specifically rhinitis, which can be allergic or non-allergic and is often associated with infection and prevents normal nasal function. By way of example, allergic and non-allergic inflammation of the nasal airway can typically effect between 10 and 20% of the population, with nasal congestion of the erectile tissues of the nasal concha, lacrimation, secretion of watery mucus, sneezing and itching being the most common symptoms. As will be understood, nasal congestion impedes nasal breathing and promotes oral breathing, leading to snoring and sleep disturbance. Worryingly, the incidence of such allergic and non-allergic inflammatory diseases is increasing. Other nasal conditions include nasal polyps which arise from the paranasal sinuses, hypertrophic adenoids, secretory otitis media, sinus disease and reduced olfaction.

In the treatment of certain nasal conditions, the topical administration of medicaments is preferable, particularly where the nasal mucosa, especially the olfactory region and distribution into the mucosa innervated by the trigeminal nerve in high drug substance concentration for rapid diffusion into the brain is the prime pathological pathway, such as in treating or relieving CNS injury, disease or disorders. Indeed, topical administration is advantageous in minimizing the possible side effects of systemic administration. Pharmaceutical compositions that are commonly topically delivered include decongestants, antihistamines, cromoglycates, steroids, including C-20 steroids, and antibiotics.

There are now an increasing number of adults and children who rely on pharmaceuticals to relieve symptoms associated with nasal conditions. At present, among the known anti-inflammatory pharmaceuticals, topical steroids have been shown to have an effect on nasal congestion. Topical decongestants have also been suggested for use in relieving nasal congestion. The treatment of hypertrophic adenoids and chronic secretory otitis media using topical decongestants, steroids and anti-microbial agents, although somewhat controversial, has also been proposed. Further, the topical administration of pharmaceuticals has been used to treat or at least relieve symptoms of inflammation in the anterior region of the nasopharynx, the paranasal sinuses and the auditory tubes.

Furthermore, medicaments are now increasingly systemically delivered through the nasal pathway, the nasal pathway offering a good administration route for the topical, systemic and CNS delivery of pharmaceuticals, such as steroids, including C-20 steroids, hormones, for example oxytocin, and anti-migraine compositions, as the high blood flow and large surface area of the nasal mucosa advantageously provides for rapid systemic uptake.

Preferably, the pharmaceutical composition is for the treatment of any one or more of the above-mentioned conditions. By way of example, the pharmaceutical composition may be for the treatment of any allergic and non-allergic inflammatory disease.

Typical pharmaceutical compositions for nasal administration in accordance with the present invention include, but are not limited to, steroids, *e.g*., C-20 steroids, anti-histamines, cromoglycates, anti-allergic pharmaceuticals, anti-inflammatory pharmaceuticals, anti-leukotrienes, lactation promoters, such as oxytocin, and anti-migraine pharmaceuticals.

By achieving a more optimal and, optionally broad drug substance spectrum, nasal delivery, the nasal insufflator art through the discovery of a novel single-directional insufflator or breath-powered nasal device and methods improves the effect of topical pharmaceuticals in the preferable treatment of CNS injury, disease or disorder, especially TBI, and most preferably concussion.

It should be understood that any reference, document, patent, patent publication or the like cited or disclosed herein, including articles and websites, are incorporated herein by reference in their entireties as if fully set forth herein.

The present disclosure has been described with reference to example embodiments. Modifications and alterations will occur to others of skill in the art upon reading and understanding the preceding detailed description. It is intended that the present disclosure be construed as including all such modifications and alterations insofar as they come within the scope of the appended claims or the equivalents thereof.

### DESCRIPTION OF NUMERICAL LEGENDS

single-directional insufflator or breath-powered nasal device **10**
nose guard **20**
   nose guard seat **22**
   through-hole **24**
      top **25**
      bottom **26**
dual wall drug dispensing system **30,** i.e, a double walled-tubing configuration
   top end portion **31**
   bottom end portion **32**
   two hollow chambers **33**
      inner hollow chamber **33a**
         outer wall **331**
         inner wall **332**
      outer hollow chamber **33b**
         outer wall **333**
         inner wall **334**
   separate chambers **34**
   rib walls **35**
   internal gap **36**
mesh disc **40**
one-way valve **50** (not shown)
first tube connector **60**
   first seat **61**
   first top hollow collar **65**
   first bottom hollow neck **66**
second tube connector **70**
   second seat **71**
   second top hollow collar **75**
   second bottom hollow neck **76**
corrugated tubing **80**
   first hollow cuff **81**
      first top end **811**
   second hollow cuff **82**
      Seconds top end **821**
   corrugated middle section **85**
   top view **850**
   bottom view **860**
one-way valve mouth piece **100**
   conduit **101**
   top section **102**
   bottom section **103**
bridge connector **110**
   bridge **121**
   first hollow ring **122**
   second hollow ring **123**
   perforation or score line **124**
nose guard side **130**
one-way valve mouth piece side **131**
front view of single-directional insufflator or breath-powered nasal device **135**
back view of single-directional insufflator or breath-powered nasal device **136**
drug substance **200** (not shown)
U-shaped configuration **140**
seal cap **150,** i.e., top cover
   seal cap plug **151**
first air-flow directional indicator band **160**
second air-flow directional indicator band **170**
alternative connector **180**

The following is a list of embodiments of the invention:
(1) A novel single-directional breath-powered nasal device for providing dual airflow for propelling a drug substance, when the drug substance is loaded into said device, into a nasal cavity for targeted delivery to the olfactory region in high drug substance concentration for rapid diffusion into the brain for the treatment of local or systemic nasal and/or central nervous system ("CNS") injury, disease or disorder, said device comprises:
   (a) a mouse piece for blowing a breath into said device by a subject;
   (b) a nose guard for propelling the drug substance in the nasal cavity of the subject;
   (c) a flexible convoluted middle section sandwiched between said mouth piece and nose guard; and
   (d) a dual wall drug dispensing system comprised of a first hollow means into which the drug substance is loaded that generates a first airflow, and a second hollow means that surrounds the first hollow means, so that, when a subject blows air through said mouthpiece and into said device, said first hollow means and said second hollow means in parallel provide a dual airflow comprised of (i) a first airflow expelled from said first hollow means carrying the drug substance, and (ii) a second airflow expelled from the second hollow means to create airflow pressure that surrounds the first expelled airflow, so that the first expelled airflow forms and travels initially in a vertical, somewhat tight and narrow or confined, concentrated plume that drives the concentrated drug substance to the targeted olfactory region for direct diffusion into the brain for the effective treatment.
(2) A device of embodiment 1, wherein the drug substance is deposited into the superior nasal cavity.
(3) A device of embodiment 1, wherein the drug substance is distributed into mucosa innervated by the trigeminal nerve.
(4) A device of embodiment 1, wherein the CNS injury is brain injury.
(5) A device of embodiment 1, wherein the brain injury is traumatic brain injury ("TBI").
(6) A device of embodiment 1, wherein the TBI is mild TBI, moderate TBI or severe TBI.
(7) A device of embodiment 4, wherein the brain injury is a concussion.
(8) A device according to embodiment 1, wherein the drug substance is *ent-19-*norprogesterone.
(9) A device according to embodiment 2, wherein the drug substance is *ent-19-*norprogesterone.
(10) A device according to embodiment 3, wherein the drug substance is *ent-19-*norprogesterone.
(11) A device according to embodiment 4, wherein the drug substance is ent-19-norprogesterone.
(12) A device according to embodiment 5, wherein the drug substance is *ent-19-*norprogesterone.
(13) A device according to embodiment 6, wherein the drug substance is *ent-19-*norprogesterone.
(14) A device according to embodiment 7, wherein the drug substance is *ent-19-*norprogesterone.
(15) A method of treating a subject for local or systemic nasal injury, disorder or disease or CNS injury, disorder or disease, who is in need of treatment, said method comprises:
   (a) using the device of embodiment 1 to deliver a therapeutically effective concentrated amount of drug substance to the olfactory region of a subject for rapid diffusion into the brain for the treatment of the local or systemic nasal injury, disorder or disease and/or CNS injury, disease or disorder.
(16) A method of embodiment 9, wherein the concentrated drug substance is deposited into the superior nasal cavity.
(17) A method of embodiment 9, wherein the concentrated drug substance is distributed into mucosa innervated by the trigeminal nerve.
(18) A method of embodiment 9, wherein the CNS injury is brain injury.
(19) A method of embodiment 9, wherein the brain injury is traumatic brain injury ("TBI").
(20) A method of embodiment 9, wherein the TBI is mild TBI, moderate TBI or severe TBI.
(21) A method of embodiment 12, wherein the brain injury is a concussion.
(22) A method of embodiment 9, wherein the drug substance is *ent-19-norprogesterone.*
(23) A method of embodiment 10, wherein the drug substance is *ent-19-norprogesterone.*
(24) A method of embodiment 11, wherein the drug substance is *ent-19-norprogesterone.*
(25) A method of embodiment 12, wherein the drug substance is *ent-19-norprogesterone.*
(26) A method of embodiment 13, wherein the drug substance is *ent-19-norprogesterone.*
(27) A method of embodiment 12, wherein the drug substance is *ent-19-norprogesterone.*
(28) A method of embodiment 13, wherein the drug substance is *ent-19-norprogesterone.*
(29) A method of embodiment 14, wherein the drug substance is *ent-19-norprogesterone.*
(30) A method of embodiment 15, wherein the drug substance is *ent-19-norprogesterone.*
(31) A method of embodiment 16, wherein the drug substance is *ent-19-norprogesterone.*
(32) A method of embodiment 17, wherein the drug substance is *ent-19-norprogesterone.*
(33) A method of embodiment 18, wherein the drug substance is *ent-19-norprogesterone.*
(34) A method of embodiment 19, wherein the drug substance is *ent-19-norprogesterone.*
(35) A method of embodiment 20, wherein the drug substance is *ent-19-norprogesterone.*
(36) A method of embodiment 21, wherein the drug substance is *ent*-19-norprogesterone.

## Claims

1. An *ent*-19-norprogesterone drug substance for use in a method of treating a subject for local or systemic nasal injury, disorder or disease or CNS injury, disorder or disease, wherein the *ent*-19-norprogesterone drug substance is delivered using a single-directional breath-powered nasal device to the olfactory region of the subject, wherein said device comprises:
(a) a mouth piece for blowing a breath into said device by the subject;
(b) a nose guard for propelling the drug substance in the nasal cavity of the subject;
(c) a flexible convoluted middle section sandwiched between said mouth piece and nose guard; and
(d) a dual wall drug dispensing system comprised of a first hollow means into which the drug substance is loaded that generates a first airflow, and a second hollow means that surrounds the first hollow means, so that, when a subject blows air through said mouthpiece and into said device, said first hollow means and said second hollow means in parallel provide a dual unidirectional airflow comprised of (i) a first airflow expelled from said first hollow means carrying the drug substance, and (ii) a second airflow expelled from the second hollow means to create airflow pressure that surrounds the first expelled airflow, so that the first expelled airflow forms and travels initially in a unidirectional, somewhat tight and narrow or confined, concentrated plume that drives the concentrated drug substance to the targeted olfactory region for direct diffusion into the brain for the effective treatment.

2. The *ent-*19-norprogesterone drug substance for use according to claim 1, wherein the drug substance is deposited into the superior nasal cavity.

3. The *ent*-19-norprogesterone drug substance for use according to claim 1 or 2, wherein the drug substance is distributed into mucosa innervated by the trigeminal nerve.

4. The *ent-*19-norprogesterone drug substance for use according to any one of claims 1 to 3, wherein the CNS injury is brain injury.

5. The *ent-*19-norprogesterone drug substance for use according to claim 4, wherein the brain injury is traumatic brain injury ("TBI").

6. The *ent-*19-norprogesterone drug substance for use according to claim 5, wherein the TBI is mild TBI, moderate TBI or severe TBI.

7. The *ent-*19-norprogesterone drug substance for use according to claim 4, wherein the brain injury is a concussion.
